(19) Europäisches Patentamt
European Patent Office
Office européen des brevets

(11) Numéro de publication: **0 208 599 B1**

# (12) FASCICULE DE BREVET EUROPEEN

(45) Date de publication du fascicule du brevet: **30.08.89**

(51) Int. Cl.⁴: **C07H 21/00**

(21) Numéro de dépôt: **86401409.7**

(22) Date de dépôt: **26.06.86**

(54) **Nouveaux supports, leur préparation et les intermédiaires obtenus, leur application à la synthèse d'oligonucléotides et les nouveaux nucléosides et oligonucléotides reliés aux supports ainsi obtenus.**

(30) Priorité: **27.06.85 FR 8509786**

(43) Date de publication de la demande:
**14.01.87 Bulletin 87/3**

(45) Mention de la délivrance du brevet:
**30.08.89 Bulletin 89/35**

(84) Etats contractants désignés:
**BE CH DE FR GB IT LI LU NL SE**

(56) Documents cités:
**ZEITSCHRIFT FÜR CHEMIE, vol. 23, no. 9, septembre 1983, pages 317-327, Leipzig, DE; A. ROSENTHAL et al.: "Chemische Synthese von DNA-Sequenzen"**
**Affinity Chromatography, J. Turkova, pages 176, 260, 261, 274, 275, 292, 293 (Elsevier 1978)**
**The Proteins (Tome II) (Academic Press 1976), pages 294-7, 300, 301**

(73) Titulaire: **ROUSSEL-UCLAF, 35, boulevard des Invalides, F-75007 Paris(FR)**

(72) Inventeur: **Buendia, Jean, 3, Impasse Emilie, F-94170 - Le Perreux Sur Marne(FR)**
Inventeur: **Nierat, Jeanine, 44, rue Carnot, F-92150 - Suresnes(FR)**

(74) Mandataire: **Bourgouin, André et al, Département des Brevets ROUSSEL UCLAF B.P. no 9, F-93230 Romainville(FR)**

EP 0 208 599 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

L'invention concerne de nouveaux supports, la préparation de ces supports, les intermédiaires nouveaux ainsi obtenus, leur application à la synthèse d'oligonculéotides et les nouveaux nucléosides et oligonucléotides reliés aux supports ainsi obtenus.

Des supports contenant des groupements sulfonamides sont décrits dans: The proteins, volume II, 1976, page 294–297/300–301 et dans: Affinity Chromatography, J. Turkova (Elsevier 1978) (J. of Chromatography Library tome 12), pages 176, 260, 261, 274, 275, 292 et 293.

Ces supports ne sont pas stables et ne sont pas utilisables pour la synthèse d'oligo-nucléotides.

De nombreux supports ont déjà été décrits dans la littérature pour la synthèse en phase solide d'oligonucléotides.

Ces supports sont par exemple uniquement constitués de polymères, tels que le polystyrène (Nucleic. Ac. Res. 1980, volume 8), le polyacrylamide acryloylmorpholide, le polydimethylacrylamide polymérisé sur kieselguhr (Nucleic Ac. Res. 9 [7] 1691 [1981]) de formule kieselguhr polyacrylamide

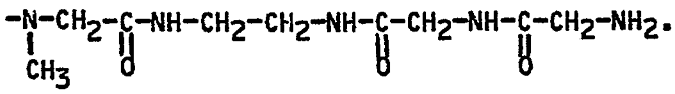

Ces supports se sont révélés insuffisants, car ils ont tendance à gonfler excessivement et à retenir certains réactifs.

D'autres supports déjà décrits sont de nature inorganique. On peut citer, par exemple, le support:

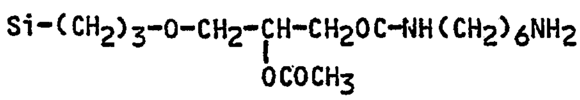

(J. Am. Chem. Soc., 105, 661 (1983)) ou le support à base de silice fonctionnalisée par un groupement 3-aminopropyltriéthoxysilane, dont l'utilisation en synthèse phosphite et phosphoramidite pour la préparation d'oligo-nucléotides a été décrite pour la première fois dans le brevet européen N° 0 035 719.

Cependant, ce support utilisé en synthèse phosphotriester donne de mauvais rendements, notamment lors des premiers couplages.

L'invention a pour objet des supports de formule (I):

$$\text{Ⓟ}-(CH_2)_m-\left[NH-CO-(CH_2)_{x_1}\right]_x-NH-\left[SO_2-(A)_{y_1}-NH\right]_y-SO_2-(A)_{y_1}-NH_2$$

dans laquelle Ⓟ est un matériau constitué de microbilles de verre fonctionnalisées, de silice fonctionnalisée par des groupements aminoalkyltrialkoxysilane de façon à obtenir pour Ⓟ la valeur

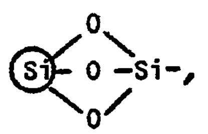

de kieselguhr, de polytétrafluoroéthylène, d'oxydes métalliques ou de cellulose,

- m est un nombre entier pouvant varier de 1 à 20,
- $x_1$ est un nombre entier pouvant varier de 1 à 20,
- x est un nombre entier pouvant varier de 0 à 20,
- A représente soit une chaîne alkyle linéaire ou ramifiée renfermant de 1 à 20 atomes de carbone, soit un radical cyclique saturé renfermant de 3 à 12 atomes de carbone, soit un radical phényle, soit un radical hétérocyclique renfermant 5 ou 6 chaînons,
- $y_1$ est un nombre entier pouvant varier de 1 à 10,
- et y est un nombre entier pouvant varier de 0 à 20.

Dans la formule (I), lorsque A est une chaîne alkyle linéaire, A est représenté par le groupement $-(CH_2)_n-$, n étant un nombre entier pouvant varier de 1 à 20.

Lorsque A est une chaîne alkyle ramifiée, il s'agit de préférence d'une chaîne substituée par un ou plusieurs radicaux méthyle ou éthyle. Il s'agit, par exemple, de la chaîne méthyl-1 méthane diyl; méthyl-1 éthane diyl-1,2; méthyl-1 ou -2 propane diyl-1,3; méthyl-1,2 propane diyl-1,3; éthyl-1 éthane diyl-1,2.

Lorsque A représente un radical cyclique saturé, il s'agit de préférence du radical cyclopropane, cyclobutane, cyclopentane cyclohexane, cycloheptane, cyclooctane, cyclononane, cyclodécane, cycloundécane, cyclododécane.

Lorsque A représente un radical hétérocyclique renfermant 5 ou 6 chaînons, il s'agit de préférence du radical thiazolyle, pyridinyle, 4,5-dihydrothiazolyle, oxazolyle, isoxazolyle, imidazolyle, pyrimidyle, thiényle.

Par "fonctionnalisées" dans le cas des microbilles de verre, on entend que les groupements en question comportent une fonction amino terminale qui permet une fixation aisée des motifs contenant les fonctions sulfonamides.

L'invention concerne notamment les supports de formule (I) répondant à la formule :

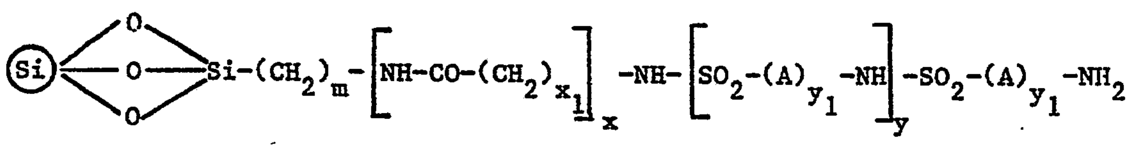

ainsi que les supports de formule (I) répondant à la formule :

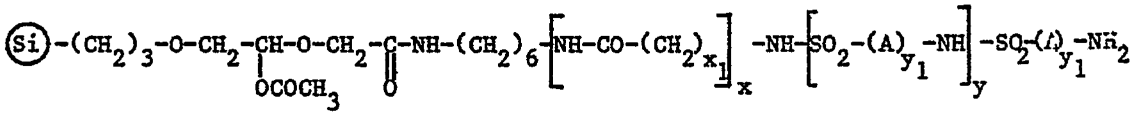

formules dans lesquelles m, $x_1$, x, $y_1$, A, y sont définis comme précédemment.

L'invention concerne aussi notamment des supports de formule (I) pour lesquels ® est un support ayant une granulomètrie homogène.

On peut utiliser une silice commerciale, par exemple la silice VYDAC A® ayant des grains dont le diamètre est de 20μm et des pores de 300 Å.

Toute autre silice comparable à la silice VYDAC A® peut être utilisée.

On peut employer également une silice pour chromotagraphie, une silice HPLC, par exemple la silice commercialisée sous le nom de porosil B®, dont le diamètre des grains est compris entre 37 et 75 μ.

L'invention concerne notamment des nouveaux supports de de formule (I), pour lesquels ® est un support ayant une granulométrie homogène et pour lesquels m est un nombre entier pouvant varier de 1 à 10 et pour lesquels $x_1$ est un nombre entier pouvant varier de 1 à 10 et x est un nombre entier pouvant varier de 0 à 10 et plus particulièrement de nouveaux supports de formule (I) pour lesquels A est un radical $-CH_2-$ ou un radical phényle, ceux pour lesquels $y_1$ est un nombre entier pouvant varier de 1 à 5 et y est un nombre entier pouvant varier de 0 à 10 et ceux pour lesquels, dans le cas où ® est un groupement

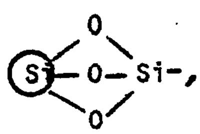 , m = 3.

L'invention a tout particulièrement pour objet des supports dont les formules suivent:

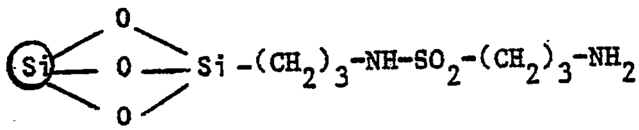

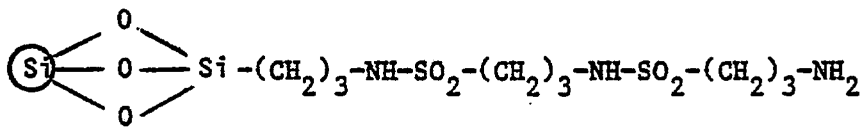

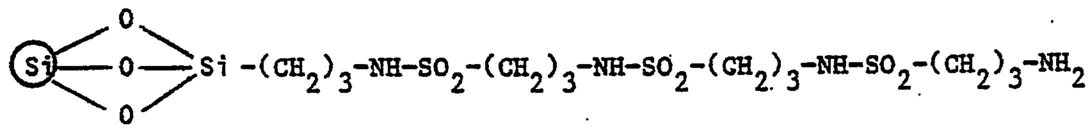

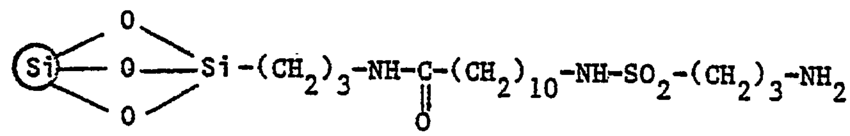

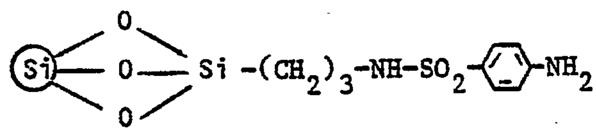

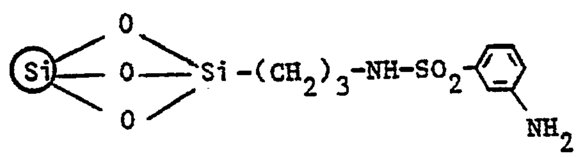

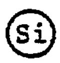 étant une silice VYDAC A® ou une silice équivalente, ainsi que le 35 support de formule:

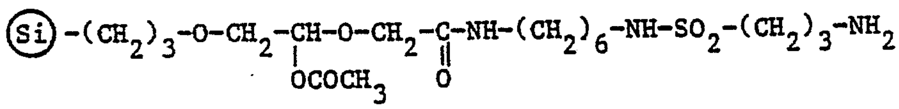

(dérivé du verre C.P.G./LCAA ou Controlled Pore Glass Long Chain Alkyl 40 Amine®).

L'invention concerne aussi un procédé de préparation des supports de formule (I) caractérisé en ce que l'on fait réagir un réactif de formula (II) :

$$RN-(A)_{y_1}-SO_2Cl \qquad (II)$$

R étant un groupement protecteur monovalent ou divalent de la fonction amine, A et $y_1$ ayant les significations précédentes, en présence d'une base aminée tertiaire,

soit avec un support de formule (III) :

$$(P)-(CH_2)_m-NH_2 \qquad (III)$$

dans laquelle ® et m ont les significations données précédemment, pour obtenir un support intermédiaire de formule :

$$(P)-(CH_2)_m-NH-SO_2-(A)_{y_1}-NR$$

dont on libère le groupement $-NH_2$ terminal, pour obtenir ainsi un support de formule (I) dans laquelle ®, m, A, $y_1$ ont les significations précédentes et dans laquelle x = o et y =o, support que l'on traite de nouveau, le cas échéant, avec un produit de formule (II), dans les mêmes conditions que précédemment, pour obtenir un support intermédiaire de formule :

$$(P)-(CH_2)_m-NH-SO_2-(A)_{y_1}-NH-SO_2-(A)_{y_1}-NR$$

dont on libère de nouveau le groupement $-NH_2$ terminal pour obtenir ainsi un support de formule (I), dans laquelle x = o et y = 1, procédé que, le cas échéant, l'on continue ainsi de suite, en passant par des supports intermédiaires de formule :

$$(P)-(CH_2)_m-NH-\left[SO_2-(A)_{y_1}\right]_y-SO_2(A)_{y_1}-NR$$

dans laquelle R, ®, m, A, $y_1$ ont les significations précédentes et dans lesquelles y est un nombre entier pouvant varier de 2 à 20, jusqu'à obtention, si désiré, d'un support de formule (I), dans laquelle x = o et y = 20,

soit avec un support de formule (IV) :

$$(P)-(CH_2)_m-\left[NH-CO-(CH_2)_{x_1}\right]_x-NH_2$$

dans laquelle ®, m, et $x_1$ ont les significations données précédemment et x est un nombre entier pouvant varier de 1 à 20, pour obtenir un support intermédiaire de formule :

$$(P)-(CH_2)_m-\left[NH-\underset{\underset{O}{\|}}{C}-(CH_2)_{x_1}\right]_x-NH-SO_2-(A)_{y_1}-NR$$

dont on libère le groupement $-NH_2$ terminal pour obtenir ainsi un support de formule (I) dans laquelle ®, m, $x_1$, A et $y_1$ ont les significations précédentes et dans laquelle x est un nombre entier pouvant varier de 1 à 20 et y = O, support que l'on traite de nouveau, le cas échéant, avec un produit de formule (II) dans les mêmes conditions que précédemment, pour obtenir un support intermédiaire de formule :

$$(P)-(CH_2)_m-\left[NH-\underset{\underset{O}{\|}}{C}-(CH_2)_{x_1}\right]_x-NH-SO_2-(A)_{y_1}-NH-SO_2-(A)_{y_1}-NR$$

dont on libère le groupement $NH_2$ terminal pour obtenir un support de formula (I) dans laquelle x est un nombre entier pouvant varier de 1 à 20 et y = 1 et procédé que, le cas échéant, l'on continue en passant par des supports intermédiaires de formule :

$$(P)-(CH_2)_m-\left[NH-\underset{\underset{O}{\|}}{C}-(CH_2)_{x_1}\right]_x-NH-\left[SO_2-(A)_{y_1}-NH\right]_y-SO_2-(A)_{y_1}-NR$$

dans laquelle R, ®, m, $x_1$, A et $y_1$ ont les significations précédentes et dans lesquelles x est un nombre entier pouvant varier de 1 à 20 et y est un nombre entier pouvant varier de 2 à 20, lusqu'à obtention, si désiré, d'un support de formule (I) dans laquelle y = 20.

Dans le réactif de formule II, le groupement protecteur de la fonction amine R est, par exemple, un radical acyle dérivant d'un acide carbonique tel que le radical éthoxy-carbonyle, benzyloxy-carbonyle, tertbutyloxycarbonyle (=BOC), paraméthyloxy-benzyl oxy-carbonyle, fluorényl méthoxy carbonyle (= FMOC), un radical phtalimido, ou un radical formant un dérivé azoïque, par exemple un dérivé de formule:

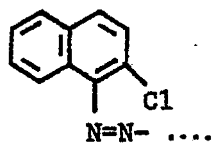

On peut aussi utiliser d'autres radicaux tels que les radicaux aryle ou aralkyle substitués ou non, par exemple, les radicaux benzyle ou triphényl méthyl ou o-nitro phényl sulfényle.

Dans les conditions préférentielles de mise en oeuvre du procédé de l'invention :

- Le support de formula III ou IV est soumis à l'action d'un réactif de formule (II) pour lequel R est un groupement protecteur de type FMOC ou un groupement azoïque de formule :

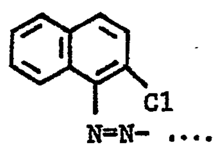

- La réaction entre le support de formule (III) ou (IV) avec le réactif (II) s'effectue en présence d'un solvant chloré, tels que le chlorure de méthylène ou le chloroforme et d'une base tertiaire telle que le pyridine ou la triéthylamine.
- Les conditions de déblocage de la fonction amine terminale des supports intermédiaires sont variables suivant les groupes protecteurs utilisés. Ainsi, par exemple :
- Lorsque R est un groupement FMOC, on utilise une amine de forte basicité, telle que la pipéridine; d'autres amines, telles que la pyrrolidine ou les dialcoylamines peuvent aussi être employées ;
- lorsque l'on utilise le groupement phtalamido, on utilise de préférence l'hydrate d'hydrazine ;
- lorsque l'on utilise un groupement azoïque tel que celui qui a été mentionné plus haut, on utilise de préférence l'hydrosulfite de soude en présence d'hydroxyde de sodium.

Les conditions de déblocage de la fonction amine terminale dans le cas des autres radicaux mentionnés précédemment, sont connues de l'homme de métier.

On prépare les réactifs de formule (II) pour lesquels R est un groupement protecteur FMOC, selon le schéma réactionnel suivant : (J.O.C. Vol 57, 22, 1972) :

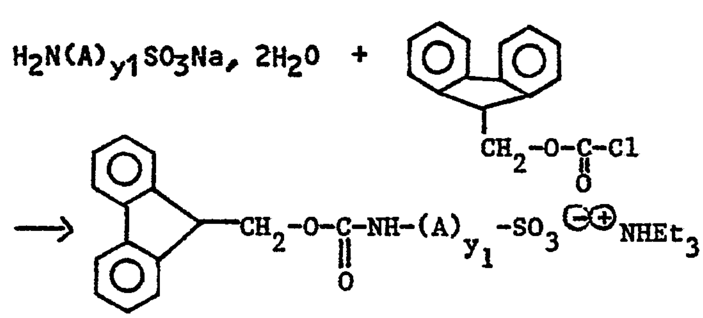

Pour obtenir le réactif II, on fait réagir le produit ainsi obtenu avec le chlorure de thionyle.

Dans le cas où R représente un radical phtalimido, on opère par exemple selon la technique décrite dans Synthesis 739, (1976).

Dans le cas où R représente un groupement azoïque tel que celui qui a été mentionné précédement, on opère par exemple selon la technique décrite dans Teilheimer 17, 559, p. 227.

D'une manière générale, les méthodes de protection des amines évoquées précédemment sont bien connues de l'homme de métier.

Les supports de formule III utilisés comme certains produits de départ dans le procédé de l'invention sont préparés comme indiqué dans le brevet européen n° 0035719.

Les supports de formule IV utilisés comme autres produits de départ dans le procédé de l'invention sont préparés selon le procédé décrit dans Chemistry Letters p. 1597-1600, 1983.

L'invention concerne aussi à titre produits industriels nouveaux, les supports de formule :

$$\text{Ⓟ}-(CH_2)_m-\left[NH-\underset{\underset{O}{\|}}{C}-(CH_2)_{x_1}\right]_x-NH-\left[SO_2-(A)_{y_1}-NH\right]_y-SO_2-(A)_{y_1}-NR$$

dans laquelle ®, m, $x_1$, x, $y_1$, y, A et R sont définis comme précédemment.

Les supports de formule (I) de l'invention permettent de réaliser, sans inconvénient, la synthése d'oligonucléotides.

L'invention a donc pour objet l'application des supports de formule (I) dans la synthèse en phase solide d'oligonucléotides par la méthode aux phosphoramidites, aux phosphites, aux phosphodiesters et aux phosphotriesters.

Ils permettent notamment d'obtenir un titre élevé en premiers nucléosides et des intermédiares stables. Ils peuvent être utilisés facilement dans les méthodes les plus classiques de synthèse en phase solide d'oligonucléotides (méthode au phosphoramidite, au phosphite, au phosphodiester, au phosphotriester), aussi bien en 3'→5' qu'en 5'→3' et pour toutes les bases puriques ou pyrimidiques usuelles.

Par ailleurs, l'hydrolyse finale séparant le support de l'oligonucléotide est réalisée facilement et en même temps que la déprotection des liaisons phosphates et des groupements protecteurs des bases puriques et pyrimidiques.

L'invention concerne tout particulièrement l'application des supports de formule (I) dans la synthèse solide par la méthode aux phosphotriesters et par la méthode aux phosphoramidites.

L'invention aussi pour objet les désoxyribonucléosides et ribonucléosides obtenus lors de la synthèse desoligonucléotides mettant en jeu les support de formule (I).

L'invention a ainsi pour objet de nouveaux désoxyribonucléosides et ribonucléosides sur supports de formule :

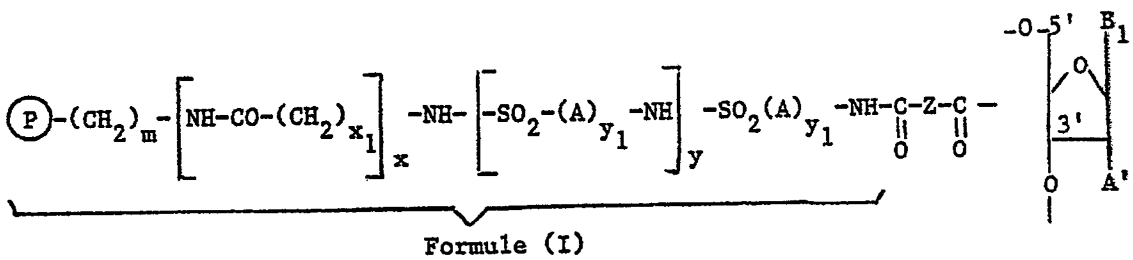

Formule (I)

dans laquelle le support de formule (I) est relié à un ribonucléoside ou à un désoxyribonucléoside, soit en 3', soit en 5', par l'intermédiaire d'un groupement

$$-\underset{\underset{O}{\|}}{C}-Z-\underset{\underset{O}{\|}}{C}-,$$

Z étant un groupement hydrocarboné renfermant de 2 à 20 atomes de carbone, ou un groupement phényle, la fonction hydroxyle non reliée en 3'ou 5' au support étant éventuellement protégée et dans laquelle A' est soit un atome d'hydrogène si le support de formule (I) est relié à un désoxyribonucléoside, soit $OR_1$ si le support de formule (I) est relié à un ribonucléoside, $R_1$ étant soit un atome d'hydrogène, soit un groupement protecteur usuel de la fonction hydroxyle, $B_1$ est une base purique ou pyrimidique dont la fonction amine est éventuellement protégée. L'invention a plus particulièrement pour objet de nouveaux oligodésoxyribonucléosides de formule :

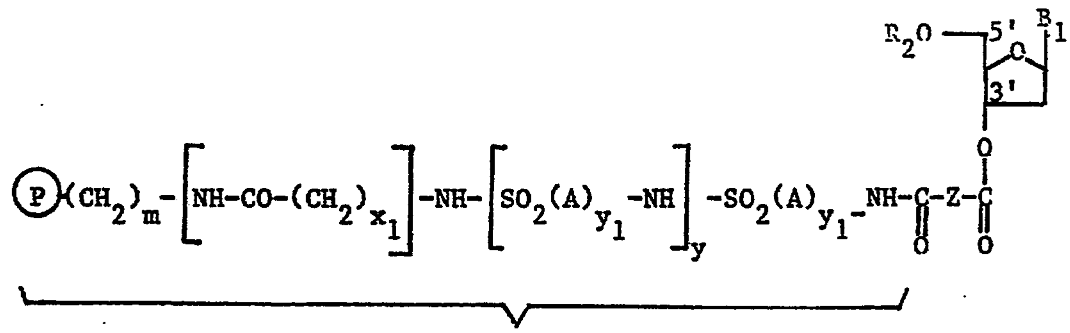

Formule (I)

dans laquelle le support de formule (I) est relié à un désoxyribonucléoside en 3' par l'intermédiaire d'un groupement

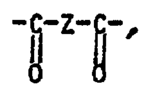

dans lequel Z est un groupement hydroxarboné renfermant de 2 à 20 atomes de carbone, et un groupement phényle, et la fonction hydroxyle en 5' est éventuellement protégée par un groupement protecteur $R_2$ usuel, $B_1$ est une base purique ou pyrimidique, dont la fonction amine est éventuellement protégée.

L'invention concerne aussi les nucléotides obtenus par application des supports de formule (I) de formule (I) à leur synthèse.

L'invention a donc pour objet de nouveaux oligodésoxyribonucléotides ou oligoribonucléotides sur supports pour lesquels le nucléoside est attaché soit en 3', soit en 5', au support de formule (I), répondant à la formule suivante :

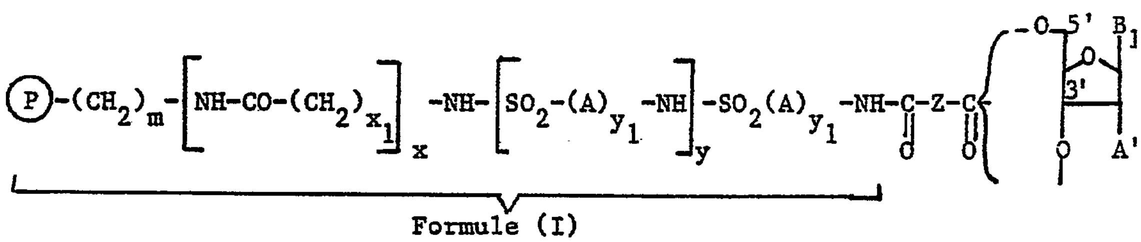

Formule (I)

et est lui-même relié par des liaisons phosphodiesters ou triesters du type:

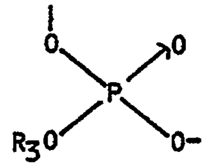

dans lesquelles $R_3$ est soit un atome d'hydrogène, sont un groupement protecteur, à d'autres nucléotides portant les bases $B_1$, ....B(z-1) jusqu'au dernier nucléoside de formule :

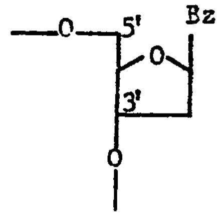

Bz étant la dernière base de l'oligodésoxy ou de l'oligoribonucléotide, la fonction hydroxyle du dernier nucléoside en 5' ou en 3' étant éventuellement protégée et les différentes bases puriques ou pyrimidiques ayant leurs fonctions amines éventuellement protégées.

L'invention a plus particulièrement pour objet de nouveaux oligodésoxyribonucléotides sur supports de formule :

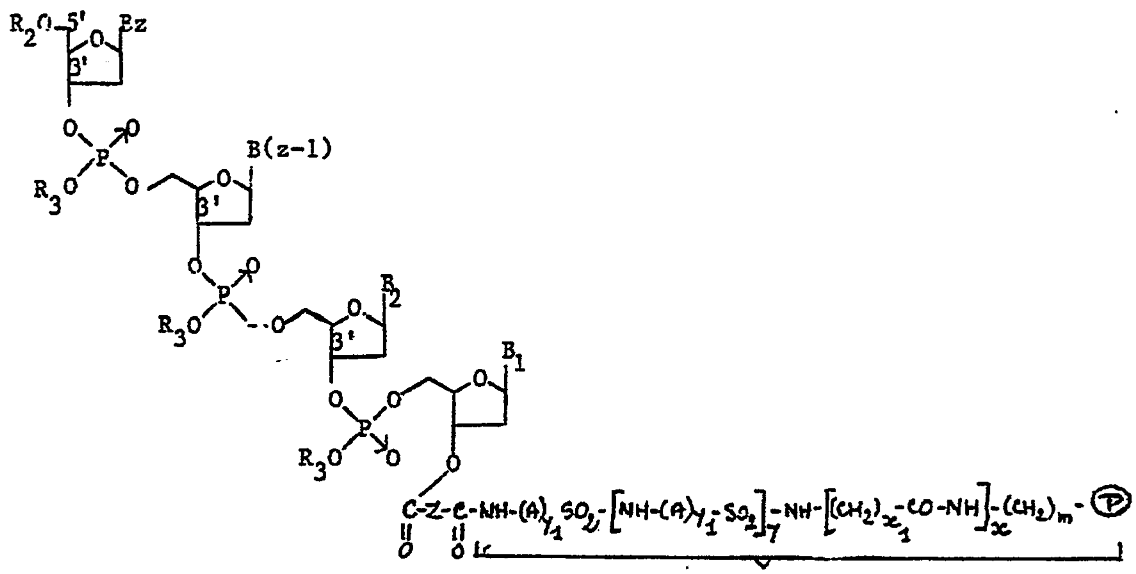

Formule (I)

dans laquelle le support de formule (I) est relié en 3' à un oligodésoxyribonucléotide portant des bases $B_1$, $B_2$-----Bz dans laquelle $R_2$ est soit un atome d'hydrogène, soit un groupement protecteur, $R_3$ est soit un atome d'hydrogène, soit un groupement protecteur, les bases puriques ou pyrimidiques ayant leurs fonctions amines éventuellement protégées.

Dans les désoxyribonucléosides, ribonucléosides, oligodésoxyribonucléotides ou oligoribonucléotides sur supports définis précédemment, Z est de préférence un radical phényle ou un radical $-(CH_2)_n$, n étant un nombre entier pouvant varier de 2 à 20.

L'invention concerne tout particulièrement ceux pour lesquels Z est un radical $-(CH_2)_2$.

Dans les ribonucléosides ou oligoribonucléotides, définis ci-dessus, $R_1$ est un groupement protecteur usuel de la fonction hydroxyle, tel qu'un groupement pyranyle, silyle ou benzyle.

Dans les nucléosides ou nucléotides définis précédemment, les bases $B_1$, $B_2$ --- Bz représentent l'adénine, la guanine (bases puriques), la cytosine, l'uracile ou la thymine (bases pyrimidiques).

Ces bases peuvent aussi être des bases puriques ou pyrimidiques substituées telles que par exemple la 6-méthylaminopurine ou la 6-diméthyl aminopurine, la 1-méthyl guanine, la 5-méthyl-cytosine, la 5-hydroxyméthyl cytosine, le dihydrouracile.

Toutes les bases dites rares ou mineures que l'on trouve dans certains acides nucléiques peuvant être utilisées.

Les groupements protecteurs des fonctions axotées de ces bases sont par exemple des groupement benzoyle ou isobutyryle.

Le groupement protecteur $R_2$ de la fonction hydroxyle en 5' est par exemple un radical trityle, monométhoxytrityle diméthoxytrityle, pixyle.

Le groupement protecteur $R_3$ des fonctions hydroxyles des groupements phosphates est par exemple le radical méthyle, un radical cyano éthyle ou un radical ortho ou parachlorophényle.

Dans le cas de la méthode aux phosphotriesters, $R_3$ est de préférence le radical ortho ou parachlorophényl.

Dans le cas de la méthode aux phosphoramidites $R_3$ est de préférence soit un radical méthyle soit un radical cyanoéthyle.

Les méthodes de synthèse de polynucléotides mentionnées précédemment sont très usuelles et parfaitement connues de l'homme de l'art. On peut les trouver résumées par exemple dans l'article The Chemical Synthesis of DNA, Aldrichimica Acta, vol. 16, N° 3,1983.

Il est rappelé briévement ci-après les différentes étapes de la synthèse 3'→5' d'oligo désoxyribonucléotides par la méthode aux phosphotriesters et par la méthode aux phosphoramidites. Il va de soi que les étapes sont strictement les mêmes pour la synthèse d'oligodésoxyribonucléotides si la synthèse se fait en 5'→3', le groupement protecteur de l'hydroxyle en 3' devant être dans ce cas convenablement choisi.

1. <u>Préparation d'un désoxynucléoside activé</u>:

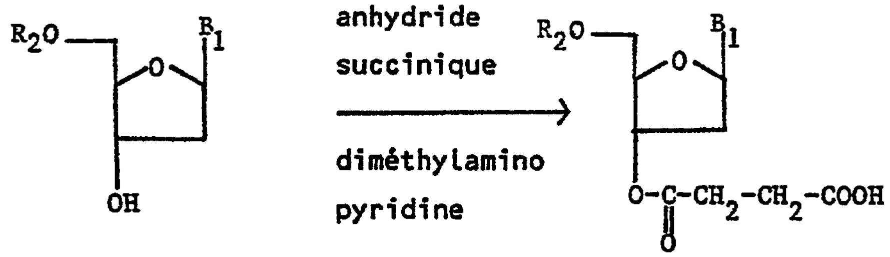


On peut utiliser l'acide libre ou activé par un groupement pentachloro phényle (Itakura et al. Nucl. Ac. Res. 8, 22, 5473, 1980) ou paranitro phényle (MH Caruthers, Chemical and Enzymatic Synthesis of gene fragments, H.Cr. Gassen et A Lang, Verlag Chemie (1982) p. 71.

2. <u>Condensation du désoxynucléoside préparé précédement sur un support de formule (1) dénommé schématiquement ci-après</u>:

Cette condensation s'effectue en solution soit dans le diméthylformamide en utilisant la triéthylamine comme catalyseur pendant 20 à 24 heures, soit dans la pyridine en présence de dicyclohexylcarbodiimide pendant une nuit à trois jours ou en présence de diméthylaminopyridine.

On obtient :

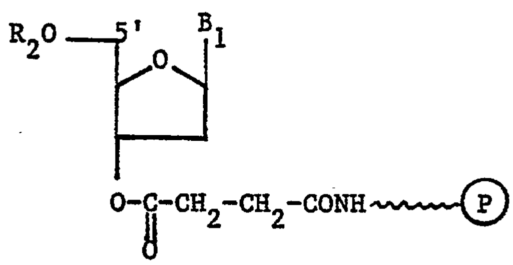

On déprotège la fonction 5' OH par traitement avec un acide de Lewis, par exemple avec le bromure de zinc ou avec un acide di ou trichloracétique.

3. <u>Allongement de la chaîne désoxyoligonucléotidique par la méthode aux phosphotriesters</u>:

On utilise soit des nucléotides monomères, soit des nucléotides dimères sous forme de leurs sels de triéthylammonium.

Les dimères sont stockés sous forme de dérivés cyanoéthylés. On prépare le sel de triéthylammonium juste avant la synthèse.

Après avoir éliminé le groupement protecteur de l'hydroxyle 5' du premier nucléoside fixé sur le support, on condense ce dimère préparé à l'étape 2 en présence d'un agent d'activation, par exemple de mésityl sulfonyle 3-nitro 1, 2, 4-triazole ou MSNT ou du mélange chlorure de mésityl sulfonyl-N-méthyle imidazole, ou encore du mélange MSNT-N-méthyl imidazole dans la pyridine.

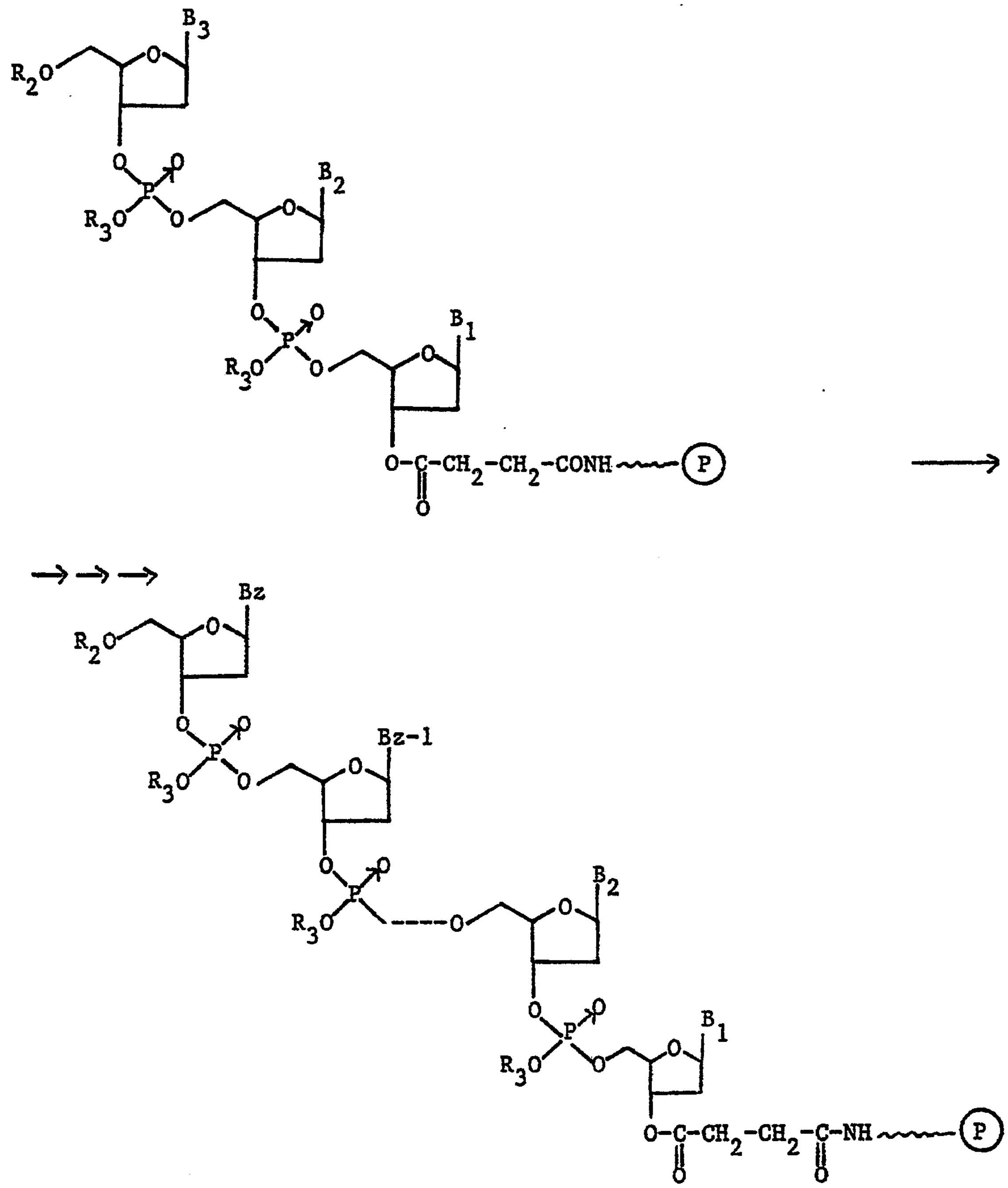

Avant chaque couplage, on élimine le groupement protecteur en 5' du dernier nucléotide accroohé sur la chaîne.

3'. <u>Allongement de la chaîne désoxyoligonucléotidique par la méthode aux phosphoramidites.</u>

Le schéma de synthèse est le suivant :

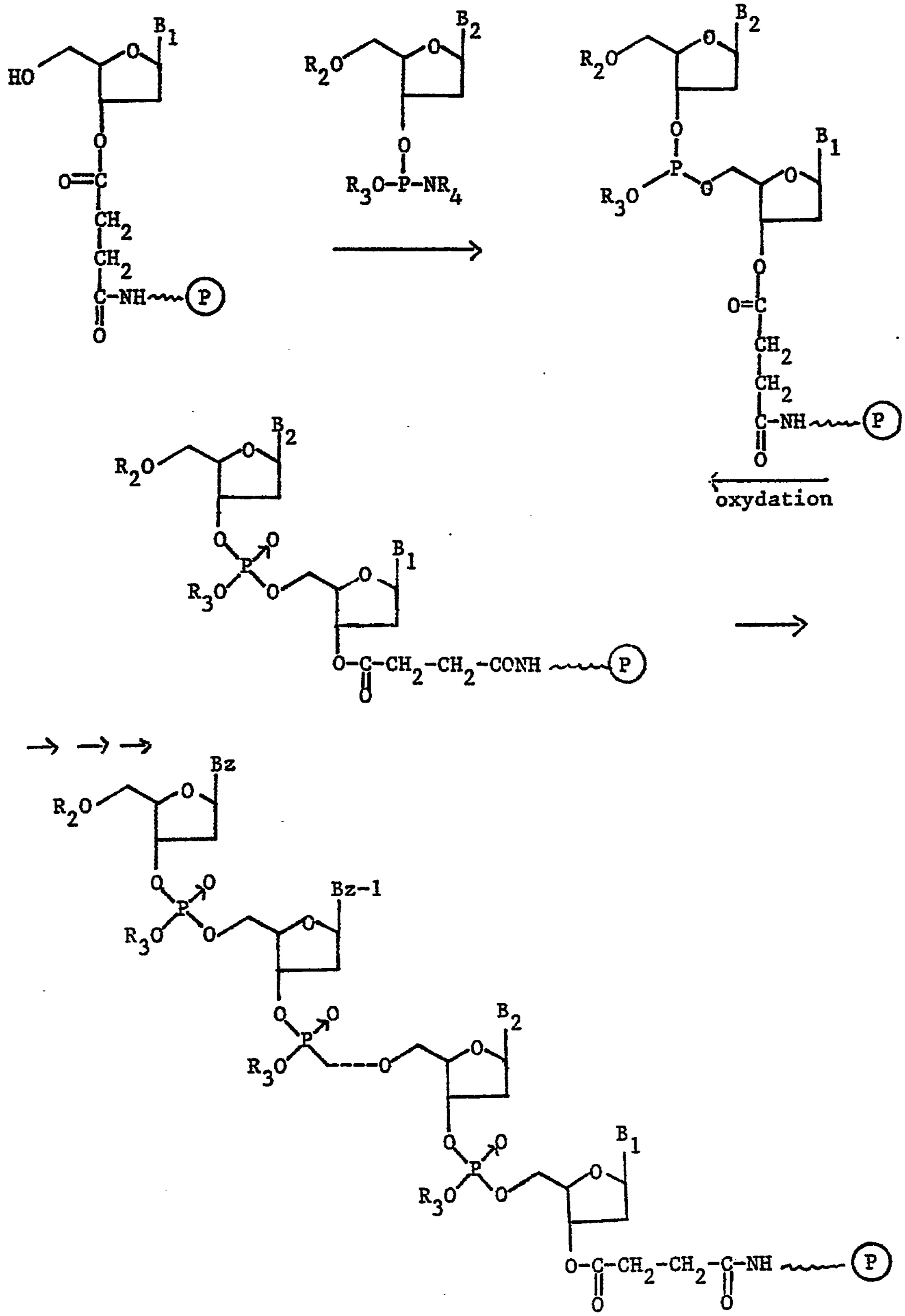

$R_4$ est un groupement divalent comme par exemple un groupement morpholino ou représente deux groupements monovalents qui sont des groupements alkyl comme par exemple isopropyl.

Les autres radicaux ont les définitions indiquées précédemment.

On utilise la technique et l'appareil commercialisé "Applied Biosystems modèle 381 A".

On fait réagir un acide sur le support de formule (I) condensé avec le premier nucléoside portant la première base $B_1$ nécessaire pour la synthèse envisagée, afin de libérer la fonction 5'-OH puis on fixe le monomère choisi, en présence de tétrazole comme agent de couplage. L'oxydation immédiate du phosphite intermédiaire obtenu conduit à un phosphate identique aux intermédiaires de la méthode aux phosphotriesters. L'allongement de la chaîne se poursuit avec la même stratégie pour les 2 méthodes.

4. <u>Déblocage et séparation du support et de la chaîne oligonucléotidique</u>.

Afin d'obtenir l'oligonucléotide déprotégé, on opère un traitement variable adapté aux différents groupements protecteurs des fonctions phosphates, amines et hydroxyles en 5'.

- Lorsque le groupement protecteur de phosphates est un radical ortho ou parachlorophényle, on utilise par exemple le mélange paranitrobenzaldoxime et N,N,N',N'-tétraméthylguanidine dans le mélange dioxane et eau 1-1.

Ce réactif très doux permet de cliver sélectivement les liaisons phosphates aryliques par rapport aux phosphates aliphatiques réalisant ainsi le déblocage des phosphates sans casser la chaîne synthétisée. On fait réagir ensuite de l'ammoniaque concentré pendant 5 ou 6 heures à 60°C pour saponifier les amides.

- Dans ce cas, on peut également utiliser uniquement de l'ammoniaque concentré en chauffant modérément le milieu réactionnel à 50°C pendant 18 à 20 heures. Le traitement unique à l'ammoniaque peut aussi être utilisé lorsque le phosphate est protégé par un radical cyanoéthyle.

- Lorsque le groupement protecteur des phosphates est un radical méthyle, on fait réagir sur le nucléotide du thiophénol à température ambiante puis on effectue le clivage des amides par l'ammoniaque concentré en chauffant modérément à 50°C pendant 18 à 20 heures comme ci-dessus.

Ces différents traitements permettent d'obtenir l'oligonucléotide déprotégé mais également de le séparer du support. Seul l'hydroxyle en 5' reste à débloquer.

Un traitement acide effectué de préférence en fin de synthèse permet la libération du groupement protecteur en 5'. On utilise l'acide acétique ou l'acide di ou trichloroacétique.

Il faut ensuite traiter l'oligonucléotide ainsi obtenu, de façon à éliminer toutes les impuretés accumulées lors de la synthèse.

Plusieurs traitements de purification (électrophorèse-chromatographies) sont alors nécessaires pour obtenir l'oligonucléotide désiré.

Seule, une ultime étape de séquençage permet de connaître de façon certaine, si celà s'avère nécessaire, la structure de l'oligonucléotide.

Les supports de formule (I) de l'invention peuvent également être appliqués à la synthèse peptidique et l'invention a donc également pour objet cette application.

Les exemples donnés ci-dessous illustrent l'invention, sans toutefois la limiter.

<u>Exemple 1</u> : Préparation du support de formule :

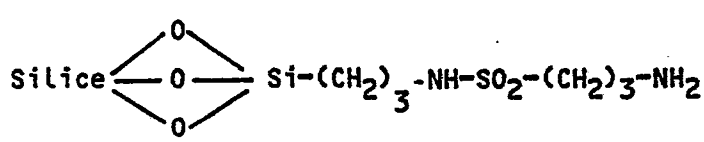

<u>STADE A</u> : Préparation du support de départ :

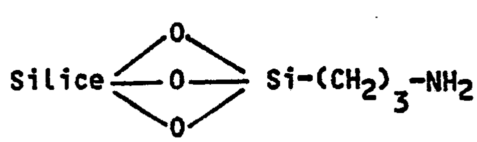

On opère comme indiqué à l'exemple 1 du brevet européen nº 0035719 à partir de 10 g de silice VYDAC A ® (granulométrie 20 μ -- Pores 300 Å) et de 11,5 g de 3-amino propyltriéthoxy silane.

On obtient le support attendu présentant un titre en $NH_2$ de $2.10^{-4}$ eq/g (dosage par la méthode à l'acide picrique).

STADE B : Préparation du support.

On dissourt partiellement 460 mg de 3 /-/(9H fluorén-9-yl) méthoxycarbonyl/amino/ propane sulfonate de N,N diéthyléthane amine (préparation donnée ci-dessous) dans 5 cm3 de chlorure de méthylène, ajoute 2 cm3 de pyridine et 0,2 cm3 de chlorure de thionyle, agite 2 heures, concentre sous pression réduite.

On ajoute à l'extrait sec obtenu 1 cm3 de pyridine, 5 cm3 de chlorure de méthylène et 500 mg de support préparé au stade A. On agite 48 Heures à l'obscurité, à température ambiante, essore, lave successivement avec du diméthylformamide puis au chlorure de méthylène, sèche sous pression réduite et obtient 500 mg de support intermédiaire ayant un titre en $NH_2$ de $0{,}28.10^{-4}$ eq/g (dosage à l'acide picrique).

On reprend avec 2 cm3 de pipéridine à 20 % le diméthylformamide, agite 30 minutes, essore, lave successivement au diméthylformamide puis au chlorure de méthylène et sèche sous pression réduite.

On obtient 450 mg de support attendu ayant un titre en $NH_2$ de $3.10^{-4}$ eq/g.

Préparation du 3-/-/(9 H fluorèn-9-yl) méthoxycarbonyl/amino/ propane sulfonate de N,N diéthyléthane amine.

On opère comme indiqué dans J. Org. Chem. Vol. 57, N° 22, 1972 à partir d'un mélange de 2,4 g de sel de sodium de l'acide aminopropane sulfonique (dihydrate) dans 19 cm3 d'eau distillée et de 4,5 g de 9-fluorénylméthylchloroformiate.

On alcalinise le milieu par 2,8 cm3 de triéthylamine, agite pendant 3 heures, amène le pH à 1 à l'aide d'acide chlorhydrique et concentre sous pression réduite.

On reprend l'extrait sec par du chlorure de méthylène, essore, lave le solide au chlorure de méthlène, concentre à sec la solution chlorométhyènique sous pression réduite et obtient 6,2 g de produit attendu.

Exemple 2 : Préparation du support de formule :

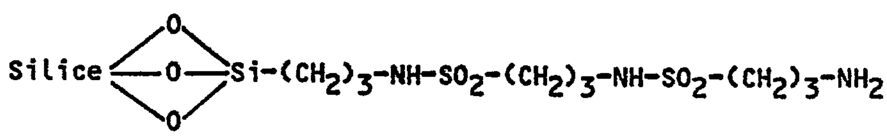

On dissout 500 mg de 3-/-/(9 H fluorèn-9-yl) méthoxy carbobyl/ amino/ propane sulfonate de N,N diéthyléthane amine dans 5 cm3 de chlorure de méthylène, 2 cm3 de pyridine, 0,2 cm3 de chlorure de thionyle.

On agite 3 heures, concentre sous pression ræduite, ajoute à l'extrait sec 5 cm3 de chlorure de méthylène, 1 cm3 de pyridine, 450 mg mg de support obtenu au stade B de l'exemple 1. On agite 72 heures à l'obscurité à température ambiante, essore, lave à l'ethanol 100° puis au chlorure de méthlène et sèche sous pression réduite. On obtient 440 mg de support intermédiaire ayant un titre en $NH_2$ de $3.10^{-5}$ eq/g. On reprend avec 2 cm3 de pipéridine à 20 % dans le diméthylformide, agite 30 minutes, essore, lave successivement au diméthylformamide puis au chlorure de méthylène et sèche sous pression réduite. On obtient 380 mg de support attendu ayant un titre en $NH_2$ de $2{,}8.10^{-4}$ eq/g.

Exemple 3 : Préparation du support de formule.

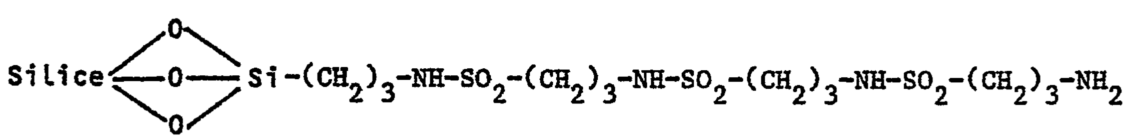

On opère comme à l'exemple 1, à partir de 500 mg de 3-/-/(9 H- fluorèn-9-yl) méthoxycarbonyl/ amino/ propane sulfonate de N, N - diéthyléthanamine et de 200 mg de support obtenu à l'exemple 2.

On obtient 200 mg de support intermédiaire ayant un titre en $NH_2$ de $0{,}3.10^{-4}$ eq/g et 190 mg de support terminal attendu ayant un titre en $NH_2$ de $2{,}3.10^{-4}$ eq/g.

Exemple 4 : Préparation du support de formule :

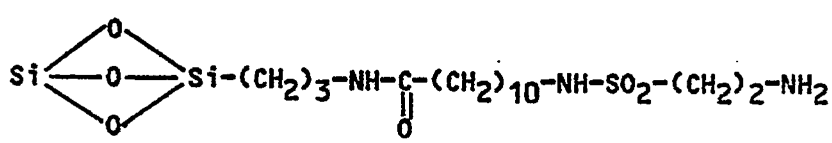

Le support de départ est un support de formule :

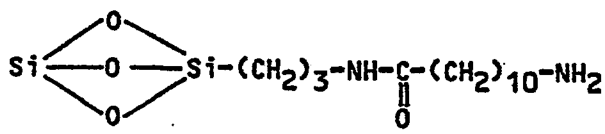

titrant 3.10$^{-4}$ eq/g en $NH_2$ et préparé selon le procédé décrit dans Chemistry Letters, p. 1597-1983 à partir du support :

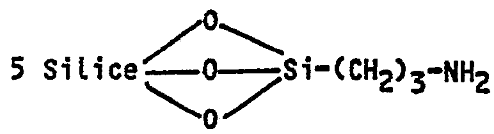

préparé au stade A de l'exemple 1.

On mélange 200 mg de support de départ avec 5 cm$^3$ de chloroforme, 1 cm3 d'acétronitrile et 300 mg de phtalimido-éthane sulfochlorure (préparation donnée ci-dessous). On ajoute ensuite, goutte à goutte, 0,28 cm$^3$ de triéthylamine; agite et porte au reflux pendant 2 heures, puis agite une nuit à température ambiante. On essore, lave au chloroforme, sèche sous pression réduite. Le test à la ninhydrine étant positif, on remet la silice on réaction dans les conditions précédentes jusqu'à ce que ce test soit négatif.

On reprend la silice avec 5 cm$^3$de méthanol, 1 cm$^3$ d'hydrate d'hydrazine à 100 %, 0,15 cm$^3$ d'eau. On agite, porte au reflux pendant 2 heures, esore, lave avec le mélange chlorure de méthylèneméthanol (1-1), sèche sous pression réduite et obtinet 185 mg de support attendu dosant en $NH_2$ : 3.10$^{-4}$ eq/g.

<u>Préparation du Phtalimido éthane sulfochlorure.</u>

On le prépare comme indiqué dans Synthesis 739 (1976) à partir de 2,9 g de sel de potassium de l'acide phtalimido éthane sulfonique, de 10 cm$^3$d'oxychlorure de phosphore et de 2 g de pentachlorure de phosphore. On obtient 1,9 g de produit attendue (PF = 162° C).

<u>Exemple 5</u> : Préparation du support de formule :

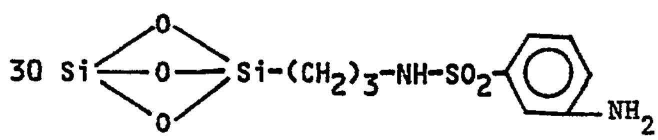

<u>Stade A</u> : Préparation du support de formule :

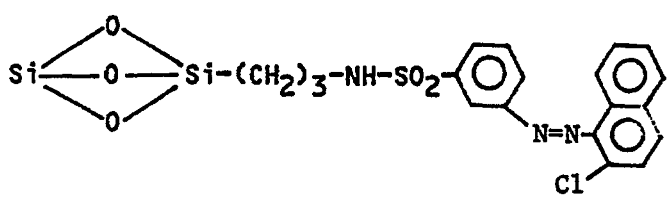

On agite pendant 24 heures 300 mg de support préparé comme au stade A de l'exemple 1, 500 mg de chlorure de l'acide 3-/(2-chloro 1- naphtalényl) axo/ benzène sulfonique, 3 cm$^3$ de chlorure de méthylène, 0,6 cm$^3$ d'acétonitrile et 0,3 cm$^3$ de triéthylamine. On essore, lave successivement avec du chlorure de méthylène puis avec du méthanol, sèche, sous pression réduite à température ambiante, et obtient 250 mg de support intermédiaire ayant un titre en $NH_2$ de 5.10$^{-6}$ eq/g.

<u>Stade B</u> : Préparation du support de formule :

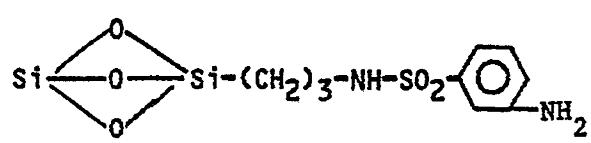

On chauffe à 50°C, 200mg du support précédent dans 5 cm3 de soude 0,1N et ajoute 1 g d'hydrosulfite de soude. On chauffe à 120°C pendant 30 minutes, refroidit, essore le support obtenu, le lave successivement à l'eau, au méthanol puis au chlorure de méthlène, le sèche sous pression réduite à température ambiante et obtient 81 mg de support attendu ayant un titre en $NH_2$ de $7,2.10^{-5}$ eq/g.

Préparation du chlorure de l'acide 3-/(2-chloro 1-naphtalényl) axo/ benzène sulfonique utilisé au départ de l'exemple 5.

Stade A : Acide -3-/(2-hydroxy 1-naphtalényl) azo/ benzène sulfonique (sel disodique).

On mélange 3,46 g d'acide métasulfanilique dans 25 cm3 d'acide chlorhydrique 2N, refroidit à O°/=2°C et ajoute goutte à goutte en 1 heure et 30 minutes 1,65 g de nitrite de soude en solution dans 8 cm3 eau. On maintient 15 minutes sous agitation et verse dans une solution préparée à température ambiante comprenant 3,17 g de β -naphtol, 22 cm3 de soude N et 1,7 g de carbonate de sodium. On agite pendant 15 minutes, essore le précipté, le lave à l'eau glacée et le sèche sous pression réduite à 80°C. On recueille 6,7 g de produit attendu.

Stade B : chlorure de l'acide 3-/(2-chloro 1-naphtalényl) axo/ benzène sulfonique.

On mélange 5,7 g de l'acide préparé au stade A, 25 cm3 d'oxychlorure de phosphore et 3,2 g de pentachlorure de phosphore. On élimine l'oxychlorure de phosphore par distillation sous pression réduite, reprend le résidu par 50 cm3 de chlorure de méthylène. On filtre, concentre à sec sous pression réduite et recristallise le résidu dans l'acétone. Après séchage à 50°C sous pression réduite, on recueille 3,25 g de produit attendu.

Spectre IR

aromatique : 1618 $^{cm-1}$
système conjugué : 1582 $^{cm-1}$ - 1503 $^{cm-1}$
$SO_2$ : 1330 $^{cm-1}$ - 1180 $^{cm-1}$ - 1170 $^{cm-1}$.

Exemple 6 : Préparation du support de formule :

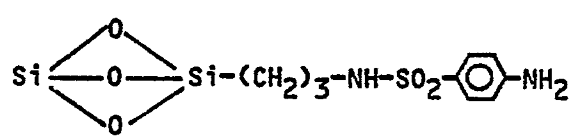

Stade A : Préparation du support de formule :

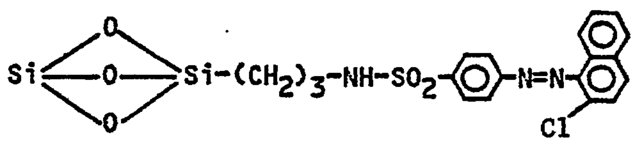

On opère comme au stade A de l'exemple 5 en utilisant au départ 400 mg de support préparé comme au stade A de l'exemple 1 et 734 mg de chlorure de l'acide 4-/(2-chloro 1-naphtalényl) axo/ benzène sulfonique. On obtient 390 mg de support intermédiaire ayant un titre en $NH_2$ de $5.10^{-6}$ eq/g et $6.10^{-6}$ eq/g.

Stade B : Préparation du support de formule :

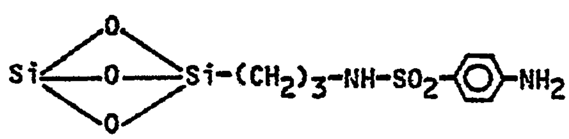

On chauffe à 50°C 200 mg de support préparé au stade A dans 10 cm3 de soude 0,1N et ajoute 1 g d'hydrosulfite de soude. On maintient sous agitation à 50°C pendant 20 minutes, essore le support obtenu, le lave successivement à l'eau, au méthanol puis au chlorure de méthylène, le sèche sous pression réduite

à température ambiante et obtient 160 mg de support attendu ayant un titre en $NH_2$ de $8.10^{-5}$ mole/g.
Préparation du chlorure de l'acide 4-/(2-chloro 1-naphtalényl) azo/ benzène sulfonique utilisé au départ de l'exemple 6.

Stade A : Acide 4-/(2-hydroxy 1-naphtalényl) axo/ benzène sulfonique (sel disodique).

On opère comme au stade A de la préparation donnée à ;a suite de l'exemple 5 en utilisant au départ 3,46 g d'acide sulfanilique dans 50 cm3 d'acide chlorhydrique N. On obtient 7,15 g de produit attendu que l'on utilise tel quel pour le stade suivant.

Stade B : chlorure de l'acide 4-/(2-naphtalényl) azo/ benzène sulfonique.

On opère comme au stade B de la préparation donnée à la suite de l'exemple 5 en utilisant 7,15 g du produit du stade A, 32 cm3 d'oxychlorure de phosphore et 8 g de pentachlorure de phosphore. On obtient 4,25 g de produit attendu.

Spectre IR

aromatiques : $1585^{cm-1}$
système conjugué : $1804^{cm-1}$
$SO_2$ : $1381^{cm-1}$ - $1181^{cm-1}$ -

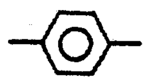 : $83^{cm-1}$.

Exemple 7 : Préparation du support de formule :

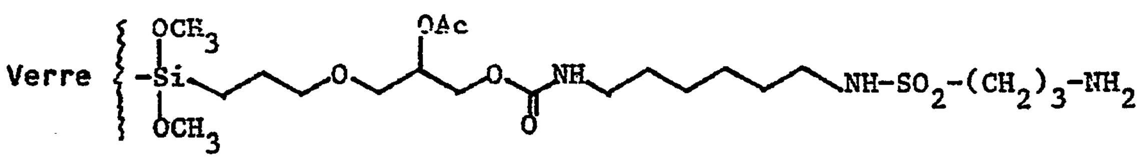

Stade A : Préparation du support de formule :

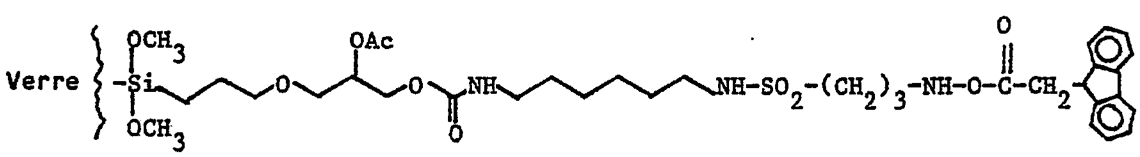

On agite pendant 12 heures 1 g de chlorure de l'acide 9-fluorényl méthyl carbamate de 3-amino propyl sulfonique obtenu dans le stade B de l'exemple 1, 4 cm3 d'acétonitrile, 200 mg de verre C.P.G. à longue chaîne alkylamine et 2 cm3 de triéthylamine. On essore, lave successivement à l'acétronitrile, puis à l'eau, puis au méthanol et enfin au chlorure de méthlène. On sèche sous pression réduite à température ambiante et obtient 190 mg de produit brut. On reprend le résidu dans 10 cm3 de pyridine avec 1 cm3 d'isocyanate de phényle puis essore, lave avec le mélange de chlorure de méthylène-méthanol (1-1) puis avec du chlorure de méthylène seul, sèche sous pression réduite à température ambiante et obtient 180 de produit attendu.

Stade B : Préparation su support :

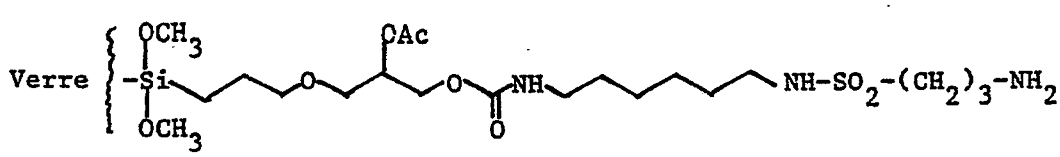

On empâte 3 fois les 180 mg de produit ci-dessus avec 1 cm3 du mélange diméthylformamide-pipéridine

(9-1). On essore, lave au méthanol puis au chlorure de méthylène, sèche sous pression réduite à température ambiante et recueille 170 mg de support attendu ayant un titre en $NH_2$ de $1,8.10^{-5}$ eq/g.

Synthèse d'oligonucléotides par la méthode au phosphotriester

Le support de formule (I) est disposeé dans une minicolonne entre deux filtres en polyfluoroéthylène. Les deux filtres sont maintenus dans une position fixe par deux pistons creux. L'ensemble est fermé en haut par un bouchon à vis muni d'un septum par lequel on introduit à la seringue le mélange de couplage. L'appareil utilisé est semblable à celui décrit dans Chemical and Enzymatic Synthésis of gene fragments H.Cr. Gassen A. Lang Verlag Chemie 82, p. 14.

Toutes les opérations répétives de lavage et d'introduction de réactifs sont automatisées. Le nombre de nucléotides à introduire pour la totalité de la synthèse peut être programmé. L'introduction du nucléotide est la seule opération manuelle à effectuer à la seringue.

On place dans le réacteur la quantité de support de formule (I) condensé avec le premier nucléoside portant la première base $B_1$ nécessaire pour la synthèse envisagèe (25 à 150 mg). On programme convenablement les paramètres souhaités, en particulier le nombre de nucléotides à accrocher successivement, puis on commence le cycle automatique suivant :

**Stade 1:**

| Solvant | Type d'introduction | Temps |
|---|---|---|
| Pyridine | Flux continu: 1 $cm^3$/mn | 5 mn |
| Isocyanate de phényle à 10% dans la pyridine | Fractions programmées | 10 mn |
| Pyridine | Flux continu: 1 $cm^3$/mn | 5 mn |
| Chlorure de méthylène | Flux continu: 2 $cm^3$/mn | 3 mn |
| Acide dichloro acétique 10% dans le chlorure de méthylène | Flux continu: 2 $cm^3$/mn | 4,5 mn |
| DMF | Flux continu: 1 $cm^3$/mn | 5 mn |
| Pyridine | Flux continu: 1 $cm^3$/mn | 5 mn |
| Couplage | à la seringue | 15 mn à 1 h |

Stade 2 :

On effectue autant de cycles identiques au premier, qu'il est nécessaire pour obtenir le nucléotide souhaité.

Les solvants utilisés dans ce cycle de synthèse doivent être très purs et anhydres.

Le dosage UV de la quantité des ions tritylium effectué après détritylation permet de connaître le rendement de chaque couplage.

Le mélange de couplage est préparé juste au moment de l'emploi, il comprend :

- 10 équivalents de sels de triéthylammonium du nucléotide monomère ou dimère (par rapport à la quantité du premier nucléoside présent sur le support solide).
- 30 équivalents de mésityl sulfonyl 3-nitro 1,2,4-triazole ou MSNT dans la pyridine anhydre (0,3 cm3 pour 50 mg de dimère environ).

Ce mélange est transféré dans une seringue sous atmosphère d'argon anhydre, on l'ajoute en trois fois à intervalles réguliers.

Pour obtenir l'oligonucléotide totalement déprotégé, il est ensuite nécessaire :

- d'effectuer un certain nombre de traitements qui sont très usuels et décrits, par exemple, dans Chemical and Enzymatic Synthesis of gene fragments H. Cr. Gassen and A. Land Verlag Chemie 82, pages 2 à 42.

Les traitements sont les suivants :

1/. Cliver le nucléotide de son support solide et déprotéger les phosphates.

On effectue un traitement par une solution 0,3 Md'o-nitro-benzaldoximate de 1, 1, 3, 3-tétraméthyl guanidinium dans un mélange dioxane-eau (1/1).

On procède de manière très usuelle, comme indiqué dans Nucleic. Acides. Research. Vol. 9 n° 18, 1981, p. 4611.

Ce réactif très doux permet ainsi de cliver sélectivement les liaisons phosphates aryliques par rapport aux phosphates aliphatiques réalisant ainsi le déblocage des phosphates sans casser la chaîne synthétisée.

2/. Débloquer les fonctions amines des bases azotées.

On effectue un traitement par $NH_4OH$ saturé ( ≃ 37%) et libère ainsi toutes les amines des bases azotées.
On peut aussi supprimer le traitement 1 et prolonger le traitement à $NH_4OH$ qui provoque les mêmes réactions.

3/. Débloquer la fonction en 5' du dernier nucléotide.

On effectue un traitement par un mélange $CH_3COOH$-eau (4-1).
Après concentration à sec, sous pression réduite, on reprend par de l'eau et extrait à l'éther pour éliminer tous les réactifs et les produits de clivage.
L'oligonucléotide à y maillons ainsi obtenu, contient de nombreuses impuretés (nucléotides à y-2, y-4-- - maillons, produits divers de dégradation).
L'obtention du produit recherché nécessite plusieurs étapes successives de purification :
- chromatoraphie sur gels,
- HPLC,
- Electrophorèse,
- Séquençage : Cette dernière méthode donne sans ambiguité, dans l'ordre, la suite des nucléotides monomères.

Ces méthodes sont très usuelles.
La demanderesse ne fait donc que les citer.

Exemple 8 : Oligo-désoxyribonucléotides synthétisés en utilisant le support de l'exemple 1.

1/. Préparation du 5'-diméthoxytrityl 2'-déoxy thymidine 3'-paranitro phényl succinate
On utilise la méthode décrite par MH Caruthers, Chemical and Enzymatic Synthetis of gene fragments, H.Cr Gassen et A Lang Verlag Chemie (1982) p. 71, à partir de 1,557 g d'acide 5'-diméthoxytrityle, 2'-déoxy thymidine 3'-succinique, de 10 cm3 de dioxane anhydre, de 0,5 cm3 de pyridine anhydre, de 369 mg de para nitrophénol, de 585 mg de dicyclohexyl carbodiimide en solution dans 2,5 cm3 de dioxane anhydre. On obtient, 1,150 g de produit attendu.
2/. Condensation entre le support de l'exemple 1 et le succinate de thymidine activé préparé ci-dessus.
On agite à température ambiante, dans l'obscurité, pendant 16 heures, 70 mg de support préparé à l'exemple 1 titrant en $NH_2$ $2.10^{-4}$eq/g, 100 mg de succinate de thymidine activé préparé ci-dessus, soit environ 11 équivalents, 150 mg de dicyclohexyl carbodiimide dans 2,5 cm3 de pyridine. On essore et lave auchlorure de méthylène, sèche sous pression réduite et obtient 60 mg de support condensé attendu ayant un titre en diméthoxytrityle de $2.10^{-5}$ eq/g.
3/. On obtient, à partir du support de l'exemple 1 condensé avec le succinate de thymidine, en utilisant trois dimères, l'oligodésoxyribonucléotide : 5'-d (TTAA AA CT).
L'agent de couplage utilisé est le M.S.N.T..

Exemple 9 : oligo-désoxyribonucléotides synthétisés en utilisant le support de l'exemple 2.

1/. Condensation entre le suppoprt de l'exemple 2 et l'acide 5'- diméthoxytrityl 2'-déoxythymidine 3'-succinique.
On agite 72 heures à température ambiante à l'obscurité 170 mg de support de l'exemple 2, 300 mg d'acide 5'-diméthoxytrityl 2'- désoxythymidine 3'-succinque, 415 mg de dicyclohexylcarbodiimide dans 3 cm3 de pyridine.
On essore, lave successivement avec de la pyridine, un mélange chlorure de méthylène-méthanol, puis avec du chlorure de méthylène. On sèche sous presion réduite et obtient 140 mg de support condensé attendu ayant un titre en diméthoxytrityle de $3,8.10^{-5}$ eq/g.
2/. On obtient à partir du support condensé préparé ci-dessus, en utilisant deux dimères en présence de -MSNT, l'oligodésoxyribonucléotide 5'-d (TTAAA).
Un deuxième est effectué en présence du mélange chlorure de mésitylène sulfonyl-N méthyl imidazole, en utilisant 3 dimères. On obtient le polydésoxy ribonucléotide 5'-d (TTA AA CT).

Exemple 10 : oligodésoxyribonucléotides synthétisés en utilisant le support de l'exemple 3.

1/. Condensation entre le support de l'exemple 3 et l'acide 5'-diméthoxytrityl 2'-désoxy thymidine 3'-succinique.
On agite 72 heures à température ambiante, à l'obscurité, 174 mg de support obtenu à l'exemple 3, 260 mg d'acide 5'-diméthoxytrityl 2'-déoxythymidine 3'-succinique, 200 mg de dicyclohexylcarbodiimide dans 3 cm3 de pyridine. On essore, lave successivement à la pyridine, au mélange chlorure de méthylène-méthanol puis au chlorure de méthylène. On sèche sous pression réduite et obtient 160 mg de support condensé attendu ayant un titre en diméthoxytrityle de $3.10^{-5}$ eq/g.

2/. On obtient à partir du support condensé préparé ci-dessus, en utilisant 2 dimdères en présence de M.S.N.T., l'oligodésoxyribonucléotide 5'-d (TTA AA).
Un deuxième essai est effectué en présence du mélange chlorure de mésitylène sulfonyle N-méthyl imidazole. On obtient l'oligo désoxyribonucléotide 5'-d (TTA AA CT).

Exemple 11 : oligodésoxyribonucléotides synthétisés en utilisant le support de l'exemple 4.

1/. Condensation entre le support de l'exemple 4 et l'acide 5'-diméthoxytrityl 2'-déoxy guanosine 3'-succinique.
On opère comme aux stades 1/ des exemples 9 et 10 et obtient le support condensé attendu ayant un titre en diméthoxytrityle de $1,1.10^{-4}$ eq/g.
2/. On obtient à partir du support condensæ préparé ci-dessus, en utilisant un monomère et un dimère l'oligodésoxy ribonucléotide 5'-d (G-ACT).
L'agent de couplage est le mélange chlorure de mésitylène sulfonyle N-méthyl imidazole.

Exemple 12 ; Oligodésoxyribonucléotides synthétisés en utilisant le support de l'exemple 5.

1/. Condensation entre le support de l'exemple 5 le succinate de thymidine activé.
On opère comme au stade 2 de l'exemple 8 en utilisant le 5'-diméthoxytrityl 2'-déoxy thymidine 3'-paranitrophényl succinate prépare au stade 1 de l'exemple 8 et le support préparé à l'exemple 5. On obtient le support condensé attendu ayant un titre en diméthoxytrityle de $2,8.10^{-5}$ eq/g.
2/. On obtient à partir du support condensé ci-dessis en utilisant 4 dimères l'oligodésoxyribonucléotide 5'-d(CATTTACTT). L'agent de couplage utilisé est le mélange MSNT-N-méthyl imidazole.

Exemple 13 : Oligodésocyribonuclétodides synthétisés en utilisant le support de l'exemple 6.

1/. Condensation entre le support de l'exemple 6 et le succinate de thymidine activé.
On opère comme au stade 2 de l'exemple 8 en utilisant la 5'-diméthoxytrityl 2'-déoxythymidine 3'-paranitrophényl succinate préparé au stade 1 de l'exemple 8 et le support préparé à l'exemple 6. On obtient le support condensé attendu ayant un titre en diméthoxytrityle de $7.10^{5}$ eq/g.
2/. En utilisant le support condensé ci-dessus et 3 dimères en présence du mélange MSNT-N-méthyl imidazole, on obtient l'oligodésoxyribonucléotide 5'-d (CATTTAT). Un deuxième essai est effectué et on obtient l'oligodésoxyribonucléotide 5'-d (TCTTCT).

Exemple 14 : Oligodésoxyribonucléotides synthétisés en utilisant le support de l'exemple 7.

1/. Préparation du 5'-diméthoxytrityl 2'-N-benzoyl désoxycytidine 3'-paranitrophényl succinate.
On utilise la méthode décrite par MH. Caruthers, Chemical and Enzymatic Synthesis of gene fragments, H-Cr Gassen et A. Lang, Verlag Chemie (1982) p. 71 à partir de 4,16 g d'acide 5'-diméthoxytrityl 2'-déoxy-N-benzoyl cytidine 3'-succinique, 25 cm3 de dioxane anhydre, 0,8 cm3 de pyridine, 1,16 g de paranitrophénol, 1,83 g de dicyclohexylcarbodiimide dans 8,33 cm3 de pyridine. On obtient 3,6 g de produit attendu.
2/. Condensation entre le support de l'exemple 7 et le succinate de cytidine activé ci-dessus.
On opère comme au stade 2 de l'exemple 8 en utilisant le succinate de cytidine activé ci-dessus et le support de l'exemple 7. On obtient le support condensé attendu ayant un titre en diméthoxytrityl de $1,5.10^{-5}$ eq/g.
3/. A partir du support condensé ci-dessus, en utilisant 3 dimères et le mélange MSNT-N-méthyl imidazole comme agent de couplage, on obtient l'oligodésoxyribonucléotide 5'-d 9CATTTAC). Un deuxième essai avec 2 dimères permet d'obtenir l'oligodésoxyribonucléotide 5'-d (TTAAC).

Exemple 15 : Oligodésoxyribonucléotides synthétisés en utilisant le support de l'exemple 1 par la méthode au phosphoramidite.

1/. Condensation entre le support de l'exemple 1 et le succinate de cytidine activé.
On opère comme au stade 2 de l'exemple 8 en utilisant le support préparé à l'exemple 1 et le succinate de cytidine activé préparé à l'exemple 14. On obtient le support condensé attendu ayant un titre en diméthoxytrytile de $1,8.10^{-5}$ eq/g.
2/. On opère selon la technique indiquée ci-dessus avec l'appareil "Applied Biosystems" en utilisant le support le support condensé du stade 1 ci-dessus et 11 monomères et en choisissant comme groupement protecteur de la fonction hydroxyle du groupement phosphate un radical méthyle. On obtient l'oligodésoxyribonucléotide suivant 5'-d 9GTACTCAGATAC). Un deuxième essai est effectué dans les mêmes conditions mais en utilisant comme groupement protecteur de la fonction hydroxyle du groupement phosphate, le radical cyanoéthyl. On obtient en utilisant 5 monomères l'oligodésoxyribonucléotide suivant 5'-d (GACTTC).

**Revendications**

1. Supports de formule (I):

$$\textcircled{P}-(CH_2)_m-\left[NH-CO-(CH_2)_{x_1}\right]_x-NH-\left[SO_2-(A)_{y_1}-NH\right]_y-SO_2-(A)_{y_1}-NH_2$$

dans laquelle Ⓟ est un matériau constitué de microbilles de verre fonctionnalisées, de silice fonctionnalisée par des groupements aminoalkyltrialkoxysilane de façon à obtenir pour Ⓟ la valeur

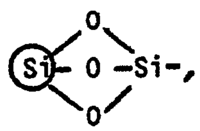

de kieselguhr, de polytètrafluoroéthylène, d'oxydes métalliques ou de cellulose,
- m est un nombre entier pouvant varier de 1 à 20,
- $x_1$ est un nombre entier pouvant varier de 1 à 20,
- x est un nombre entier pouvant varier de 0 à 20,
- A représente soit une chaîne alkyle linéaire ou ramifiée renfermant de 1 à 20 atomes de carbone, soit un radical cyclique saturé renfermant de 3 à 12 atomes de carbone, soit un radical phényle, soit un radical hétérocyclique renfermant 5 ou 6 chaînons,
- $y_1$ est un nombre entier pouvant varier de 1 à 10,
- et y est un nombre entier pouvant varier de 0 à 20.

2. Supports de formule (I) tels que définis à la revendication 1, répondant à la formule:

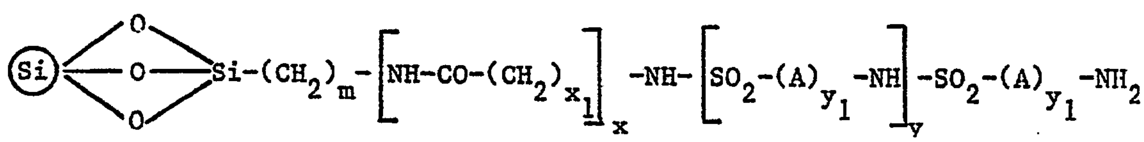

ou à la formule:

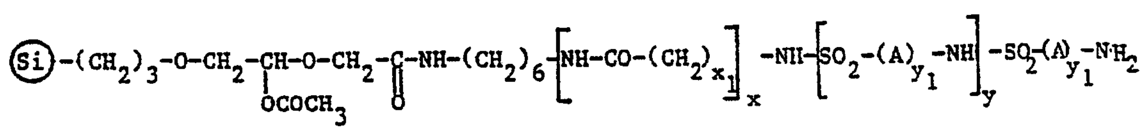

formules dans lesquelles m, $x_1$, x, $y_1$, A, y sont définis comme à la revendication 1.

3. Supports de formule (I) tels que définis à la revendication 1, pour lesquels Ⓟ est un support ayant une granulométrie homogène.

4. Supports de formule (I) tels que définis aux revendications 1 à 3, pour lesquels m est un nombre entier pouvant varier de 1 à 10.

5. Supports de formule (I) tels que définis aux revendications 1 à 4, pour lesquels $x_1$ est un nombre entier pouvant varier de 1 à 10 et x est un nombre entier pouvant varier de 0 à 10.

6. Supports de formule (I) tels que définis aux revendications 1 à 5, pour lesquels A est un radical $-CH_2-$ ou un radical phényle.

7. Supports de formule (I) tels que définis aux revendications 1 à 6, pour lesquels $y_1$ est un nombre entier pouvant varier de 1 à 5 et y est un nombre entier pouvant varier de 0 à 10.

8. Supports de formule (I) tels que définis aux revendications 1 à 7, pour lesquels Ⓟ est un groupement

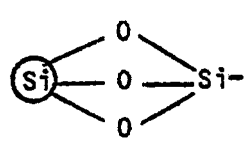

et m = 3.

9. Supports dont les formules suivent:

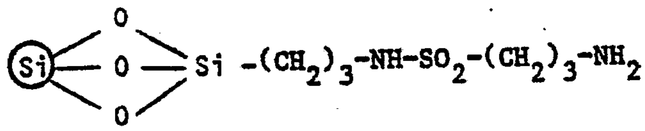

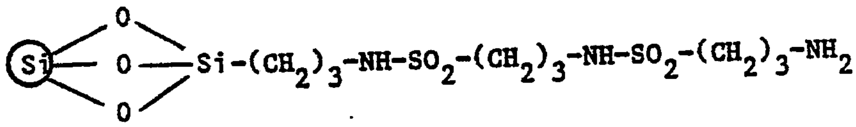

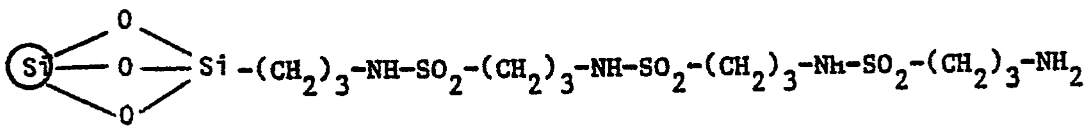

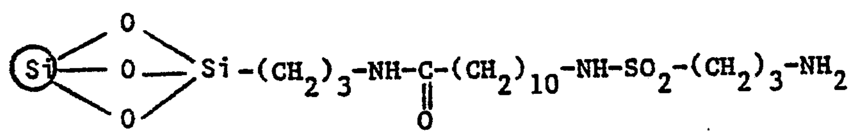

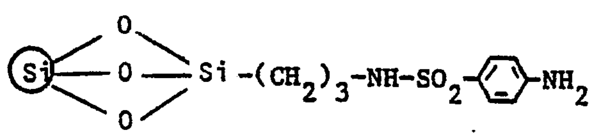

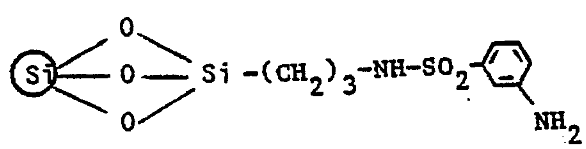

(Si) étant une silice VYDAC A® ou une silice équivalente, ainsi que le support de formule :

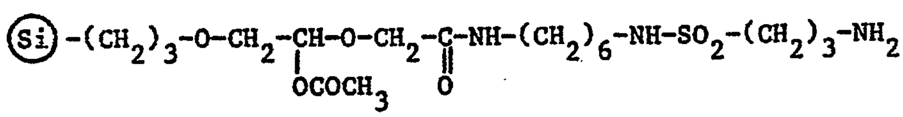

10/. Procédé de préparation des supports de formule (I) tels que définis à la revendication 1, caractérisé en ce que l'on fait réagir un réactif de formule (II) :
R-N-$(A)_{y1}$-$SO_2Cl$ (II)
R étant un groupement protecteur monovalent ou divalent de la fonction amine, A et $y_1$ ayant les significations précédentes, en présence d'une base aminée tertiaire,
<u>soit</u> avec un support de formule (III) :

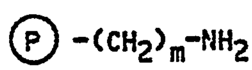 (III)

dans laquelle Ⓟ et m ont les significations données précédemment, pour obtenir un support intermédiaire de formule :

$$Ⓟ\ -(CH_2)_m-NH-SO_2-(A)_{y_1}-N-R$$

dont on libère le groupement $-NH_2$ terminal, pour obtenir ainsi un support de formule (I) dans laquelle Ⓟ, m, A, $y_1$ ont les significations précédentes et dans laquelle x = o et y =o, support que l'on traite de nouveau, le cas échéant, avec un produit de formule (II), dans les mêmes conditions que précédemment, pour obtenir un support intermédiaire de formule :

$$Ⓟ\ -(CH_2)_m-NH-SO_2-(A)_{y_1}-NH-SO_2-(A)_{y_1}-NR$$

dont on libère de nouveau le groupement $-NH_2$ terminal pour obtenir ainsi un support de formule (I), dans laquelle x = o et y = 1, procédé que, le cas échéant, l'on continue ainsi de suite, en passant par des supports intermédiaires de formule :

$$Ⓟ\ -(CH_2)_m-NH-\left[SO_2-(A)_{y_1}\right]_y-SO_2(A)_{y_1}-NR$$

dans laquelle R, Ⓟ, m, A, $y_1$ ont les significations précédentes et dans lesquelles y est un nombre entier pouvant varier de 2 à 20, jusqu'à obtention, si désiré, d'un support de formule (I), dans laquelle x = o et y = 20,
<u>soit</u> avec un support de formule (IV) :

$$Ⓟ\ -(CH_2)_m-\left[NH-CO-(CH_2)_{x_1}\right]_x-NH_2 \qquad (IV)$$

dans laquelle Ⓟ, m, et $x_1$ ont les significations données précédemment et x est un nombre entier pouvant varier de 1 à 20, pour obtenir un support intermédiaire de formule :

$$Ⓟ\ -(CH_2)_m-\left[NH-\underset{\underset{O}{\|}}{C}-(CH_2)_{x_1}\right]_x-NH-SO_2-(A)_{y_1}-NR$$

dont on libère le groupement $-NH_2$ terminal pour obtenir ainsi un support de formule (I) dans laquelle Ⓟ, m, $x_1$ A et $y_1$ ont les significations précédentes et dans laquelle x est un nombre entier pouvant varier de 1 à 20 et y = O, support que l'on traite de nouveau, le cas échéant, avec un produit de formule (II) dans les mêmes conditions que précédemment, pour obtenir un support intermédiaire de formule :

$$Ⓟ-(CH_2)_m-\left[NH-\underset{\underset{O}{\|}}{C}-(CH_2)_{x_1}\right]_x-NH-SO_2-(A)_{y_1}-NH-SO_2-(A)_{y_1}-NR$$

dont on libère le groupement $NH_2$ terminal pour obtenir un support de formule (I) dans laquelle x est un nombre entier pouvant varier de 1 à 20 et y = 1 et procédé, le cas échéant, qu l'on continue en passant par des supports intermédiaires de formule :

$$Ⓟ-(CH_2)_m-\left[NH-\underset{\underset{O}{\|}}{C}-(CH_2)_{x_1}\right]_x-NH-\left[SO_2-(A)_{y_1}-NH\right]_y-SO_2-(A)_{y_1}-NR$$

dans laquelle R, Ⓟ, m, $x_1$, A et $y_1$ ont les significations précédentes et dans lesquelles x est un nombre entier pouvant varier de 1 à 20 et y est un nombre entier pouvant varier de 2 à 20, jusqu'à obtention, si désiré, d'un support de formule (I) dans laquelle y = 20.

11/. A titre de produits industiels nouveaux, les supports de formule :

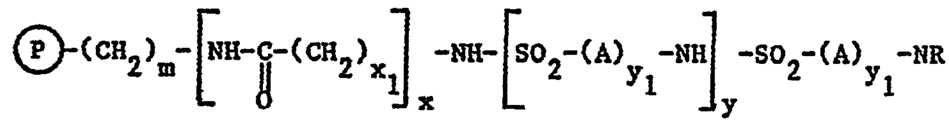

dans laquelle ®, m, $x_1$, x, $y_1$, y, A et R sont définis comme à la revendication 1.

12/. Application des supports de formule (I) tels que définis à l'une quelconque des revendications 1 à 9 dans la synthèse en phase solide d'oligonucléotides par la méthode aux phosphoramidites, aux phosphites, aux phosphodiesters et aux phosphotriesters.

13/. Application des supports de formule (I) tels que définis à l'une quelconque des revendications 1 à 9 dans la synthése solide d'oligonucléotides par la méthode aux phosphotriesters et par la méthode aux phosphoramidites.

14/. Nouveaux désoxyribonucléosides ou ribonucléosides sur supports de formule :

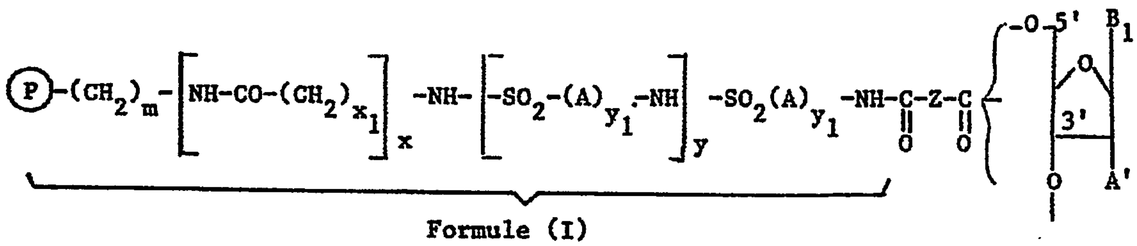

Formule (I)

dans laquelle le support de formule (I) est relié à un ribonucléoside ou à un désoxyribonucléoside, soit en 3', soit en 5', par l'intermédiare d'un groupement

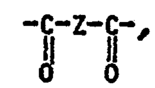

Z étant un groupement hydrocarboné renfermant de 2 à 20 atomes de carbone, ou un groupement phényle, la fonction hydroxyle non reliée en 3'ou 5' au support étant éventuellement protégée et dans laquelle A' est soit un atome d'hydrogène si le support de formule (I) est relié à un désoxyribonucléoside, soit $OR_1$ si le support de formule (I) est relié à un robonucléoside, $R_1$ étant soit un atome d'hydrogène, soit un groupement protecteur usuel de la fonction hydroxyle $B_1$ est une base purique ou pyrimidique dont la fonction amine est éventuellement protégée.

15/. Nouveaux oligodésoxyribonucléosides sur supports tels que définis à la revendication 14, de formule :

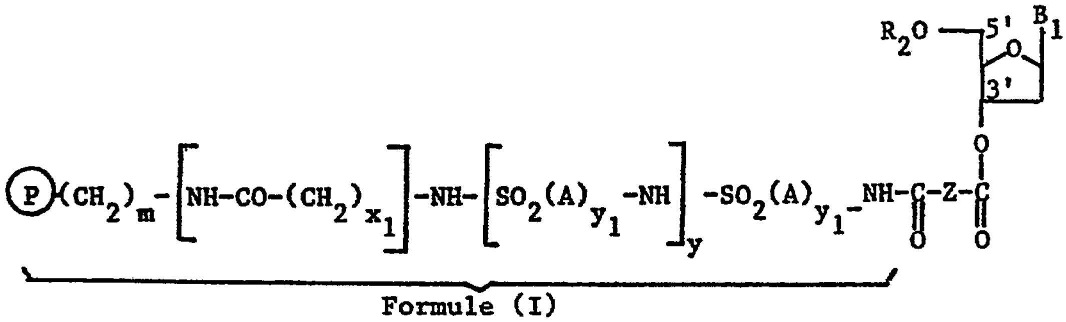

Formule (I)

dans laquelle le support de formule (I) est relié à un désoxyribonucléoside en 3' par l'intermédiaire d'un groupement

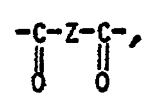

dans lequel Z est un groupement hydrocarboné renfermant de 2 à 20 atomes de carbone, ou un groupement phényle, et la fonction hydroxyle en 5' est éventuellement protégée par un groupement protecteur $R_2$ usuel, $B_1$ est une base purique ou pyrimidique, dont la fonction amine est éventuellement protégée.

16/. Nouveaux oligodésoxyribonucléotides ou oligoribonucléotides sur supports, pour lesquels le nucléoside est attaché soit en 3', soit en 5', au support de formule (I), répond à la formule suivante :

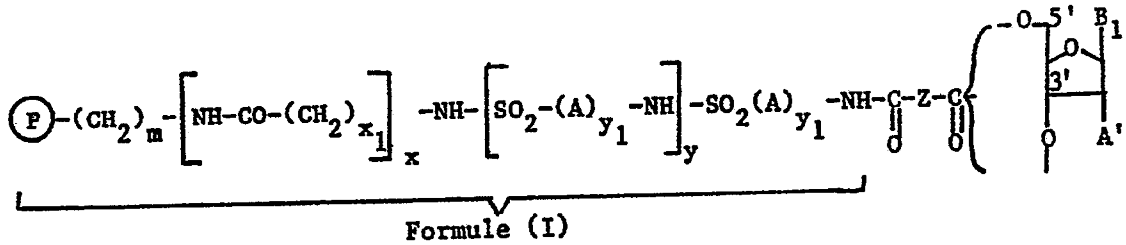

Formule (I)

et est lui-même relié par des liaisons phosphodiesters ou triesters du type :

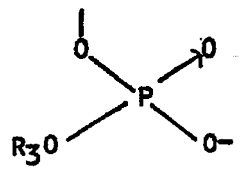

dans lesquelles $R_3$ est soit un atome d'hydrogène, soit un groupement protecteur, à d'autres nucléotides portant les bases $B_1$ .... B(z-1) jusqu'au dernier nucléoside de formule :

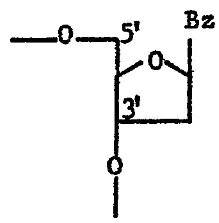

Bz étant la dernière base de l'oligodésoxy ou de l'oligoribonucléotide, la fonction hydroxyle du dernier nucléoside en 5' ou en 3' étant éventuellement protégée et les différentes bases puriques ou pyrimidiques ayant leurs fonctions amines éventuellement protégées.

17/. Nouveaux oligodésoxyribonucléotides sur supports tels que définis à la revendication 16, de formule :

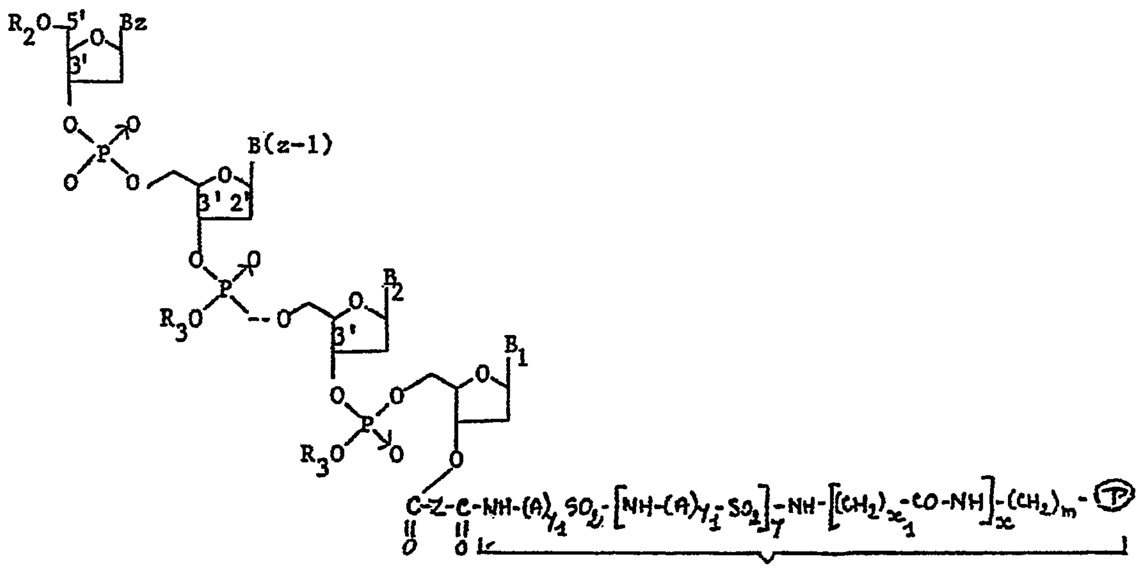

Formule (I)

dans laquelle le support de formule (I) est relié en 3' à un oligodésoxyribonucléotide portant des bases $B_1$, $B_2$-----Bz et dans laquelle $R_2$ est soit un atome d'hydrogène, soit un groupement protecteur, $R_3$ est soit un atome d'hydrogène, soit un groupement protecteur, les bases puriques ou pyrimidiques ayant leurs fonctions amines éventuellement protégées.

18/. Nouveaux oligodésoxyribonucléotides, oligorigobucléotides, désoxyribonucléosides, ribonucléosides sur supports tels que définis aux revendications 14 à 17, pour lesquels Z est le radical $-(CH_2)_2-$.

19/. Application des supports de formule (I) tels que définis à l'une quelconque des revendications 1 à 9 dans la synthèse des peptides.

**Patentansprüche**

1. Träger der Formel (I)

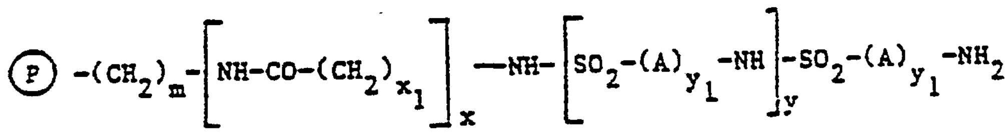

worin Ⓟ ein Material wiedergibt, bestehend aus funktionalisierten Mikrokügelchen aus Glas, aus Siliciumdioxid, funktionalisiert durch Aminoalkyltrialkoxysilan-Gruppen derart, daß man für Ⓟ die Bedeutung

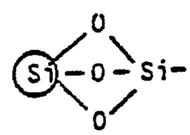

erhält, aus Kieselgur, aus Polytetrafluorethylen, aus Metalloxiden oder Cellulose,
m für eine ganze Zahl von 1 bis 20 steht,
$x_1$ für eine ganze Zahl von 1 bis 20 steht,
x für eine ganze Zahl von 0 bis 20 steht,
A entweder eine lineare oder verzweigte Alkylkette mit 1 bis 20 Kohlenstoffatomen oder einen gesättigten, cyclischen Rest mit 3 bis 12 Kohlenstoffatomen oder einen Phenylrest oder einen heterocyclischen Rest mit 5 oder 6 Kettengliedern wiedergibt,
$y_1$ für eine ganze Zahl von 1 bis 10 steht und
y für eine ganze zahl von 0 bis 20 steht.

2. Träger der Formel (I) gemäß Anspruch 1 der Formel

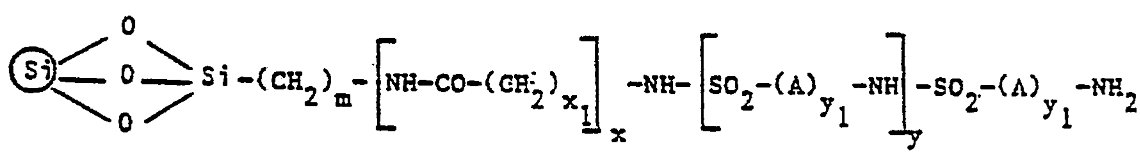

oder der Formel

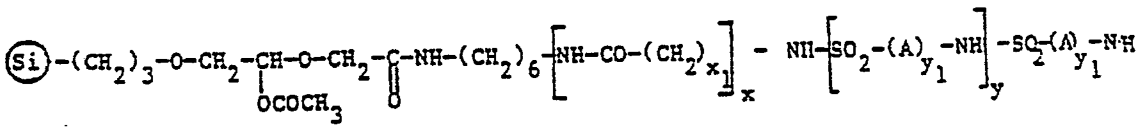

worin m, $x_1$, x, $y_1$, A und y die in Anspruch 1 angegebene Bedeutung besitzen.

3. Träger der Formel (I) gemäß Anspruch 1, worin Ⓟ einen Träger mit homogener Granulometrie wiedergibt.

4. Träger der Formel (I) gemäß den Ansprüchen 1 bis 3, worin m für eine ganze Zahl von 1 bis 10 steht.

5. Träger der Formel (I) gemäß den Ansprüchen 1 bis 4, worin $x_1$ für eine ganze Zahl von 1 bis 10 steht und x eine ganze Zahl von 0 bis 10 wiedergibt.

6. Träger der Formel (I) gemäß den Ansprüchen 1 bis 5, worin A für einen Rest $-CH_2-$ oder einen Phenylrest steht.

7. Träger der Formel (I) gemäß den Ansprüchen 1 bis 6, worin $y_1$ eine ganze Zahl von 1 bis 5 bedeutet und y für eine ganze Zahl von 0 bis 10 steht.

8. Träger der Formel (I) gemäß den Ansprüchen 1 bis 7, worin ® für eine Gruppe

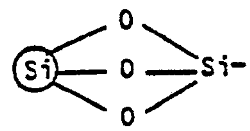

steht und m = 3.

9. Träger mit den folgenden Formeln:

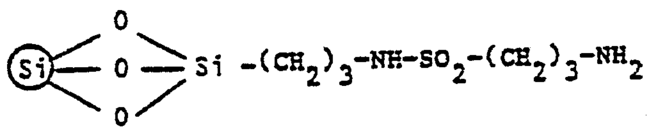

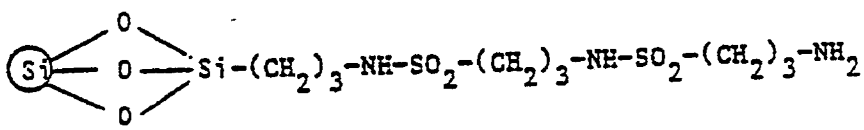

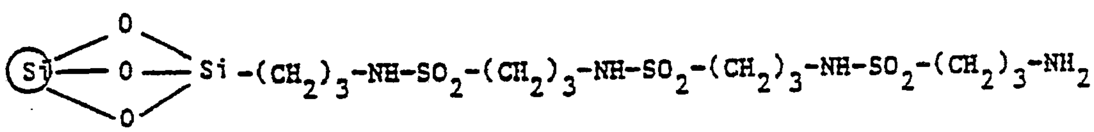

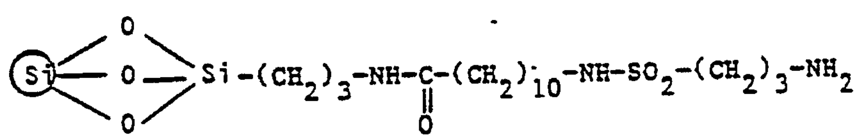

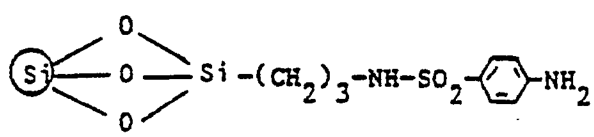

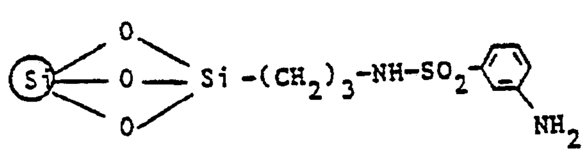

worin Si ein Siliciumdioxid VYDAC A® oder ein äquivalentes Siliciumdioxid wiedergibt, sowie der Träger der Formel

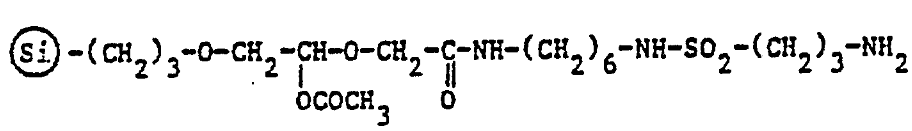

10. Verfahren zur Herstellung von Trägern der Formel (I) gemäß Anspruch 1, dadurch gekennzeichnet, daß man ein Reagens der Formel (II)

$R-N-(A)_{y_1}-SO_2Cl$ (II)
worin R eine monovalente oder divalente Schutzgruppe für die Aminfunktion wiedergibt und A und $y_1$ die vorstehenden Bedeutungen besitzen, in Gegenwart einer tertiären Aminbase <u>entweder</u> mit einem Träger der Formel (III)

$$\textcircled{P}-(CH_2)_m-NH_2 \qquad \text{(III)}$$

worin Ⓟ und m die angegebenen Bedeutungen besitzen, umsetzt, um zu einem intermediären Träger der Formel

$$\textcircled{P}-(CH_2)_m-NH-SO_2-(A)_{y_1}-N-R$$

zu gelangen, dessen endständige $-NH_2$-Gruppe man freisetzt, um so zu einem Träger der Formel (I) zu gelangen, worin Ⓟ, m, A, $y_1$ die vorstehenden Bedeutungen besitzen und worin x = 0 und y = 0, diesen Träger erneut gegebenenfalls mit einem Produkt der Formel (II) unter den gleichen Bedingungen wie vorstehend behandelt, um zu einem intermediären Träger der Formel

$$\textcircled{P}-(CH_2)_m-NH-SO_2-(A)_{y_1}-NH-SO_2-(A)_{y_1}-NR$$

zu gelangen, dessen endständige $-NH_2$-Gruppe man wiederum freisetzt, um so zu einem Träger der Formel (I) zu gelangen, worin x = 0 und y = 1, dieses Verfahren so weiter fortsetzt, wobei man über intermediäre Träger der Formel

$$\textcircled{P}-(CH_2)_m-NH-\left[SO_2-(A)_{y_1}\right]_y-SO_2(A)_{y_1}-NR$$

gelangt, worin R, Ⓟ, m, A und $y_1$ die vorstehenden Bedeutungen besitzen und worin y für eine ganze Zahl von 2 bis 20 steht, bis man gewünschtenfalls einen Träger der Formel (I) erhält, worin x = 0 und y = 20, <u>oder</u> mit einem Träger der Formel (IV)

$$\textcircled{P}-(CH_2)_m-\left[NH-CO-(CH_2)_{x_1}\right]_x-NH_2 \qquad \text{(IV)}$$

worin Ⓟ, m und $x_1$ die vorstehenden Bedeutungen besitzen und x für eine ganze Zahl von 1 bis 20 steht, umsetzt, um zu einem intermediären Träger der Formel

$$\textcircled{P}-(CH_2)_m-\left[NH-\underset{\underset{O}{\|}}{C}-(CH_2)_{x_1}\right]_x-NH-SO_2-(A)_{y_1}-NR$$

zu gelangen, dessen endständige $-NH_2$-Gruppe man freisetzt, um so zu einem Träger der Formel (I) zu gelangen, worin Ⓟ, m, $x_1$, A und $y_1$ die vorstehenden Bedeutungen besitzen und worin x für eine ganze Zahl von 1 bis 20 steht und y = 0, diesen Träger gegebenenfalls erneut mit einem Produkt der Formel (II) unter den vorstehenden Bedingungen behandelt, um zu einem intermediären Träger der Formel

$$\textcircled{P}-(CH_2)_m-\left[NH-\underset{\underset{O}{\|}}{C}-(CH_2)_{x_1}\right]_x-NH-SO_2-(A)_{y_1}-NH-SO_2-(A)_{y_1}-NR$$

zu gelangen, dessen endständige $-NH_2$-Gruppe man freisetzt, um zu einem Träger der Formel (I) zu gelangen, worin x für eine ganze Zahl von 1 bis 20 steht und y = 1, und dieses Verfahren gegebenenfalls

fortsetzt, wobei man über intermediäre Träger der Formel

$$\text{Ⓟ}-(CH_2)_m-\left[NH-\underset{\underset{O}{\|}}{C}-(CH_2)_{x_1}\right]_x-NH-\left[SO_2-(A)_{y_1}-NH\right]_y-SO_2-(A)_{y_1}-NR$$

gelangt, worin R, Ⓟ, m, $x_1$, A und $y_1$ die vorstehenden Bedeutungen besitzen und worin x für eine ganze Zahl von 1 bis 20 und y für eine ganze Zahl von 2 bis 20 steht, bis man gewünschtenfalls einen Träger der Formel (I) erhält, worin y = 20.

11. Als neue, industrielle Produkte die Träger der Formel

$$\text{Ⓟ}-(CH_2)_m-\left[NH-\underset{\underset{O}{\|}}{C}-(CH_2)_{x_1}\right]_x-NH-\left[SO_2-(A)_{y_1}-NH\right]_y-SO_2-(A)_{y_1}-NR$$

worin Ⓟ, m, $x_1$, x, $y_1$, y, A und R wie in Anspruch 1 definiert sind.

12. Verwendung der Träger der Formel (I) gemäß einem der Ansprüche 1 bis 9 bei der Synthese von Oligonucleotiden in fester Phase nach der Methode mit Phosphoramiditen, Phosphiten, Phosphodiestern und Phosphotriestern.

13. Verwendung der Träger der Formel (I) gemäß einem der Ansprüche 1 bis 9 bei der Synthese von Oligonucleotiden in fester Phase nach der Methode mit Phosphotriestern und nach der Methode mit Phosphoramiditen.

14. Neue Desoxyribonucleoside oder Ribonucleoside auf Trägern der Formel

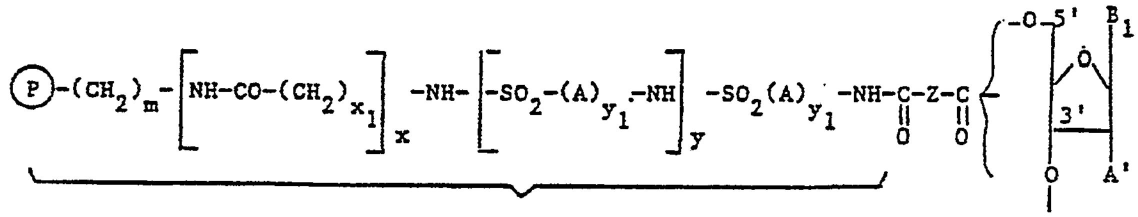

Formel (I)

worin der Träger der Formel (I) an ein Ribonucleosid oder ein Desoxyribonucleosid entweder in 3'- oder in 5'-Stellung über eine Gruppe

$$-\underset{\underset{O}{\|}}{C}-Z-\underset{\underset{O}{\|}}{C}-$$

gebunden ist, worin Z eine Kohlenwasserstoffgruppe mit 2 bis 20 Kohlenstoffatomen oder eine Phenylgruppe wiedergibt, wobei die nicht in 3'- oder 5'-Stellung an den Träger gebundene Hydroxylfunktion gegebenenfalls geschützt ist, und worin A' entweder ein Wasserstoffatom wiedergibt, wenn der Träger der Formel (I) an ein Desoxyribonucleosid gebunden ist, oder $OR_1$ bedeutet, wenn der Träger der Formel (I) an ein Ribonucleosid gebunden ist, wobei $R_1$ entweder ein Wasserstoffatom oder eine übliche Schutzgruppe für die Hydroxylfunktion bedeutet und $B_1$ eine Purin- oder Pyrimidinbase ist, deren Aminfunktion gegebenenfalls geschützt ist.

15. Neue Oligodesoxyribonucleoside auf Trägern gemäß Anspruch 14 der Formel

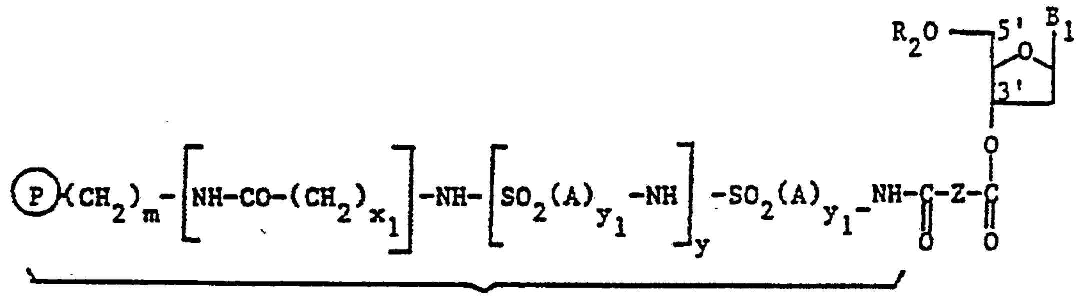

Formel (I)

worin der Träger der Formel (I) an ein Desoxyribonucleosid in 3'-Stellung über eine Gruppe

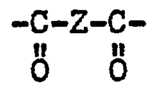

gebunden ist, worin Z eine Kohlenwasserstoffgruppe mit 2 bis 20 Kohlenstoffatomen oder eine Phenylgruppe wiedergibt, und die Hydroxylfunktion in 5'-Stellung gegebenenfalls durch eine übliche Schutzgruppe $R_2$ geschützt ist und $B_1$ eine Purin- oder Pyrimidinbase ist, deren Aminfunktion gegebenenfalls geschützt ist.

16. Neue Oligodesoxyribonucleotide oder Oligoribonucleotide auf Trägern, worin das Nucleosid entweder in 3'- oder in 5'-Stellung an den Träger der Formel (I) der folgenden Formel

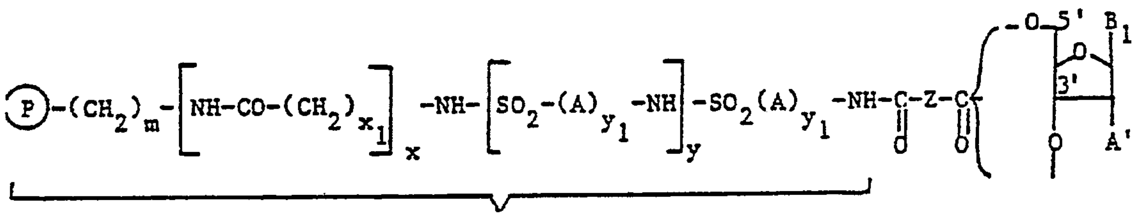

Formel (I)

gebunden ist und seinerseits durch Phosphodiester- oder -triester-Bindungen des Typs

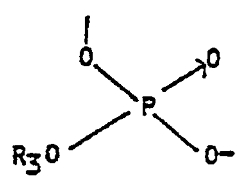

worin $R_3$ entweder ein Wasserstoffatom oder eine Schutzgruppe wiedergibt, an andere Nucleotide, die Basen $B_1$, . . . . B(z–1) aufweisen, bis zum letzten Nucleosid der Formel

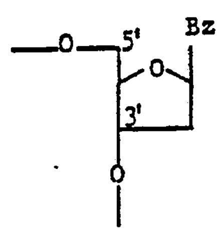

geknüpft ist, wobei Bz die letzte Base des Oligodesoxy- oder Oligoribonucleotids ist, die Hydroxylfunktion des letzten Nucleosids in 5'- oder in 3'-Stellung gegebenenfalls geschützt ist, und die Aminfunktionen der verschiedenen Purin- oder Pyrimidinbasen gegebenenfalls geschützt sind.

17. Neue Oligodesoxyribonucleotide auf Trägern gemäß Anspruch 16 der Formel

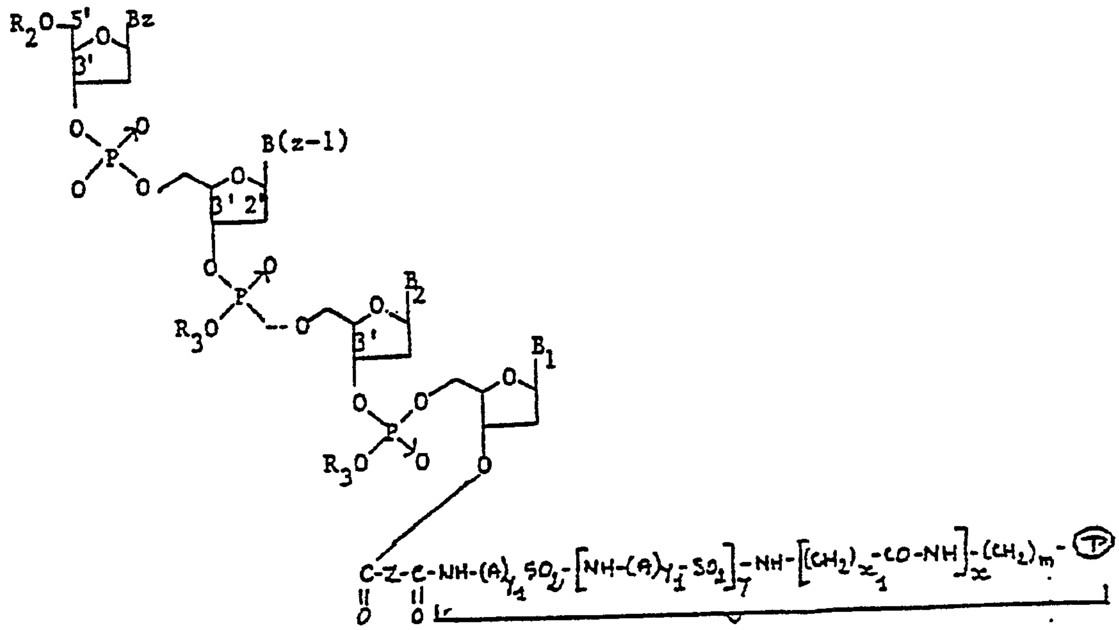

Formel (I)

worin der Träger der Formel (I) in 3'-Stellung an ein Basen $B_1$, $B_2$ ···· Bz aufweisendes Oligodesoxyribonucleotid gebunden ist und worin $R_2$ entweder ein Wasserstoffatom oder eine Schutzgruppe bedeutet, $R_3$ entweder ein Wasserstoffatom oder eine Schutzgruppe bedeutet und die Aminfunktionen der Purin- oder Pyrimidinbasen gegebenenfalls geschützt sind.

18. Neue Oligodesoxyribonucleotide, Oligoribonucleotide, Desoxyribonucleoside und Ribonucleoside auf Trägern gemäß den Ansprüchen 14 bis 17, bei denen Z den Rest $-(CH_2)_2-$ wiedergibt.

19. Verwendung der Träger der Formel (I) gemäß einem der Ansprüche 1 bis 9 bei der Peptidsynthese.

**Claims**

1. Supports of formula (I)

$$\text{Ⓟ}-(CH_2)_m-\left[NH-CO-(CH_2)_{x_1}\right]_x-NH-\left[SO_2-(A)_{y_1}-NH\right]_y-SO_2-(A)_{y_1}-NH_2$$

in which ® is a material constituted by functionalized glass microbeads, of silica made functional by aminoalkyltrialkoxysilane groups in such a way as to obtain for P the value

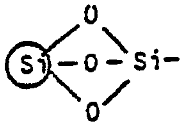

of kielselguhr, of polytetrafluoroethylene, of metallic oxides or of cellulose,
- m is an integer which can vary from 1 to 20,
- $x_1$ is an integer which can vary from 1 to 20,
- x is an integer which can vary from 0 to 20,

A represents either a linear or branched alkyl chain containing from 1 to 20 carbon atoms, or a saturated cyclic radical containing from 3 to 12 carbon atoms, or a phenyl radical, or a heterocyclic radical containing 5 or 6 ring members,
- $y_1$ is an integer which can vary from 1 to 10,

and y is an integer which can vary from 0 to 20.

2. Supports of formula (I) as defined in claim 1, corresponding to the formula:

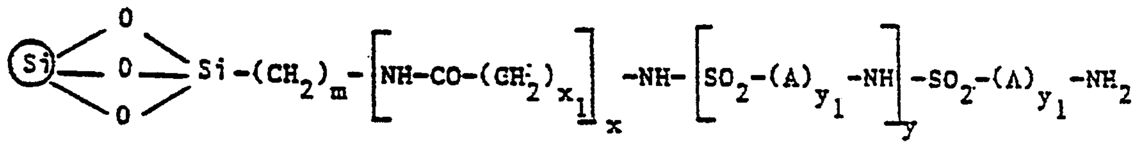

or to the formula:

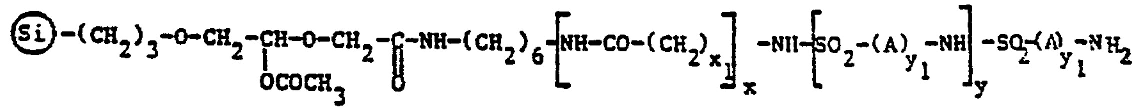

in which formulae m, $x_1$, x, $y_1$, A and y are defined as in claim 1.

3. Supports of formula (I) as defined in claim 1, for which ® is a support having a homogeneous granulometry.

4. Supports of formula (I) as defined in claims 1 to 3, for which m is an integer which can vary from 1 to 10.

5. Supports of formula (I) as defined in claims 1 to 4, for which $x_1$ is an integer which can vary from 0 to 10.

6. Supports of formula (I) as defined in claims 1 to 5, in which A is a $-CH_2-$ radical or a phenyl radical.

7. Supports of formula (I) as defined in claims 1 to 6, in which $y_1$ is an integer which can vary from 1 to 5 and y is an integer which can vary from 0 to 10.

8. Supports of formula (I) as defined in claim 1 to 7, in which ® is a

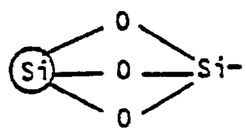

group and m = 3.

9. Supports which have the following formulae:

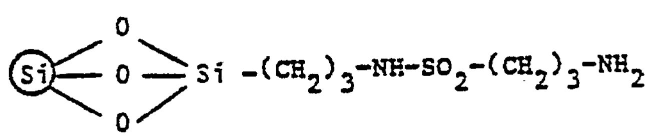

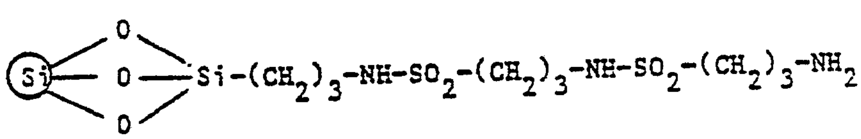

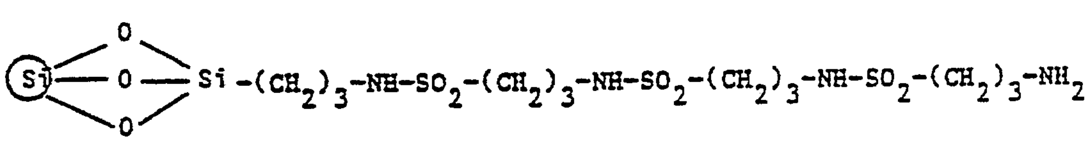

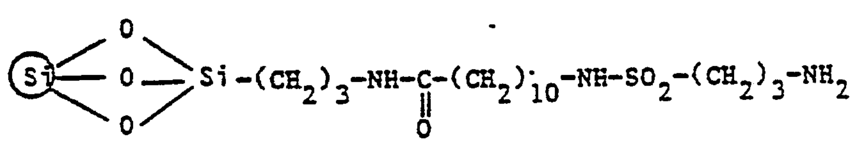

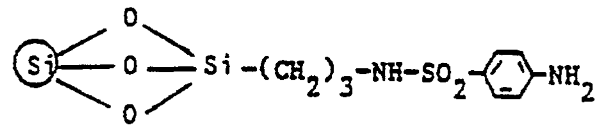

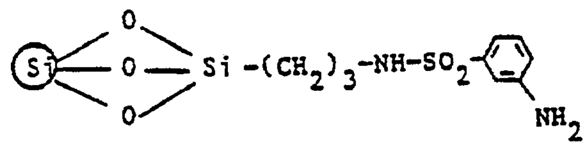

(Si) being a VYDAC A® silica or an equivalent silica, as well as the support of formula:

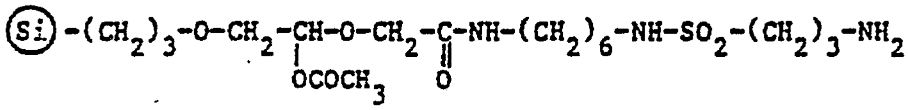

10. Process for the preparation of the supports of formula (I) as defined in claim 1, characterized in that a reagent of formula (II):

$R-N-(A)_{y_1}-SO_2Cl$ (II)

R being a monovalent or divalent protector group of the amine function, A and $y_1$ having the above meanings, is reacted in the presence of a tertiary amino base, <u>either</u> with a support of formula (III):

$$\textcircled{P}-(CH_2)_m-NH_2 \qquad (III)$$

in which ® and m have the meanings given above, to obtain an intermediate support of formula:

$$\textcircled{P}-(CH_2)_m-NH-SO_2-(A)_{y_1}-N-R$$

in which the terminal $-NH_2$ group is liberated, thus to obtain a support of formula (I) in which ®, m, A and $y_1$ have the above meanings and in which x = 0 and y = 0, which support is treated again, if necessary, with a product of formula (II), under the same conditions as before, to obtain an intermediate support of formula:

$$\textcircled{P}-(CH_2)_m-NH-SO_2-(A)_{y_1}-NH-SO_2-(A)_{y_1}-NR$$

in which the terminal $-NH_2$ is again liberated thus to obtain a support of formula (I), in which x = 0 and y = 1, which process, if necessary, is continued in the same way, passing via the intermediate supports of formula:

$$\textcircled{P}-(CH_2)_m-NH-\left[SO_2-(A)_{y_1}\right]_y-SO_2(A)_{y_1}-NR$$

in which R, ®, m, A, $y_1$ have the meanings given above and in which y is an integer which can vary from 2 to 20, until, if desired, a support of formula (I) is obtained, in which x = 0 and y = 20, <u>or</u> with a support of formula (IV):

$$\textcircled{P}-(CH_2)_m-\left[NH-CO-(CH_2)_{x_1}\right]_x-NH_2 \qquad (IV)$$

in which ®, m, and $x_1$ have the meanings given above and x is an integer which can vary from 1 to 20, to obtain an intermediate support of formula:

$$\textcircled{P}-(CH_2)_m-\left[NH-\underset{\substack{\| \\ O}}{C}-(CH_2)_{x_1}\right]_x-NH-SO_2-(A)_{y_1}-NR$$

in which the terminal $-NH_2$ group is liberated thus to obtain a support of formula (I) in which Ⓟ, m, $x_1$, A and $y_1$ have the meanings given above and in which x is an integer which can vary from 1 to 20 and y = 0, which support is treated again, if necessary, with a product of formula (II) under the same conditions as above, to obtain an intermediate support of formula:

$$\textcircled{P}-(CH_2)_m-\left[NH-\underset{\substack{\| \\ O}}{C}-(CH_2)_{x_1}\right]_x-NH-SO_2-(A)_{y_1}-NH-SO_2-(A)_{y_1}-NR$$

in which the terminal $NH_2$ group is liberated to obtain a support of formula (I) in which x is an integer which can vary from 1 to 20 and y = 1, and which process, if necessary, is continued by passing via the intermediate supports of formula:

$$\textcircled{P}-(CH_2)_m-\left[NH-\underset{\substack{\| \\ O}}{C}-(CH_2)_{x_1}\right]_x-NH-\left[SO_2-(A)_{y_1}-NH\right]_y-SO_2-(A)_{y_1}-NR$$

in which R, Ⓟ, m, $x_1$, A and $y_1$ have the above meanings and in which x is an integer which can vary from 1 to 20, until, if desired, a support of formula (I) is obtained in which y = 20.

11. As new industrial products, supports of formula:

$$\textcircled{P}-(CH_2)_m-\left[NH-\underset{\substack{\| \\ O}}{C}-(CH_2)_{x_1}\right]_x-NH-\left[SO_2-(A)_{y_1}-NH\right]_y-SO_2-(A)_{y_1}-NR$$

in which Ⓟ, m, $x_1$, x, $y_1$, y, A and R have the meanings given in claim 1.

12. Use of supports of formula (I) as defined in any of claims 1 to 9 in the synthesis, in the solid phase, of oligonucleotides by the method involving phosphoramidites, phosphites, phosphodiesters and phosphotriesters.

13. Use of the supports of formula (I) as defined in any of claims 1 to 9 in the solid synthesis of oligonucleotides by the method with phosphotriesters and by the method involving phosphoramidites.

14. New desoxyribuonucleosides or ribonucleosides on supports of formula:

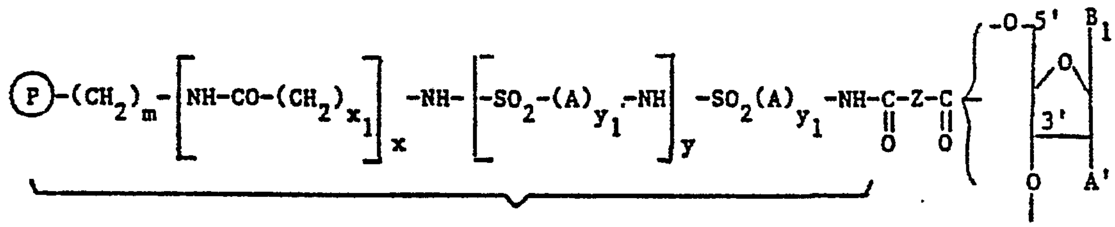

formula (I)

in which the support of formula (I) is linked to a ribonucleoside or a desoxyribonucleoside, either in position 3′ or in position 5′, via the intermediary of a

$$-\underset{\substack{\| \\ O}}{C}-Z-\underset{\substack{\| \\ O}}{C}-$$

group, Z being a hydrocarbon group containing from 2 to 20 carbon atoms, or a phenyl group, the hydroxyl function, which is not bonded in position 3′ or 5′ to the support, being optionally protected and in which A′ is either a hydrogen atom if the support of formula (I) is bonded to a desoxyribonucleoside, or $OR_1$ if the support of formula (I) is bonded to a ribonucleoside, $R_1$ being either a hydrogen atom, or a conventional protector group for the hydroxyl function, $B_1$ is a purine or pyrimidine base of which the amine function is optionally protected.

15. New oligodesoxyribonucleosides on supports as defined in claim 14, of the formula:

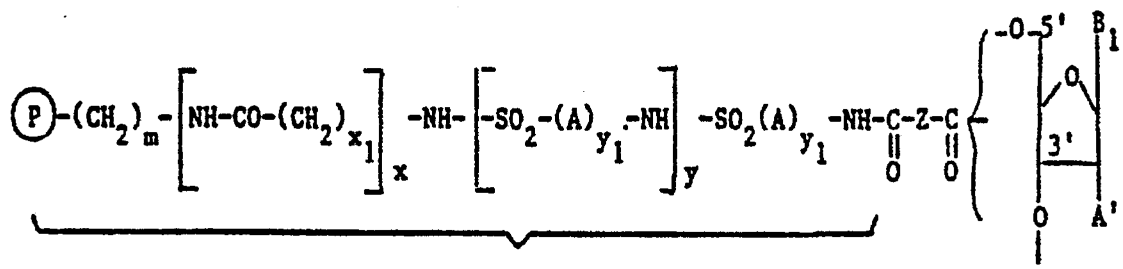

formula (I)

in which the support of formula (I) is bonded to a desoxyribonucleoside in position 3′ via the intermediary of a group

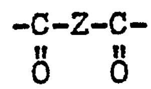

in which Z is a hydrocarbon group containing from 2 to 20 carbon atoms, or a phenyl group, and the hydroxyl function in position 5′ is optionally protected by a usual $R_2$ protector group, $B_1$ is a purine or pyrimidine base, of which the amine function is optionally protected.

16. New oligodesoxyribonucleotides or oligoribonucleotides on supports, in which the nucleoside is attached either in position 3′ or in position 5′, to the support of formula (I), correspond to the following formula:

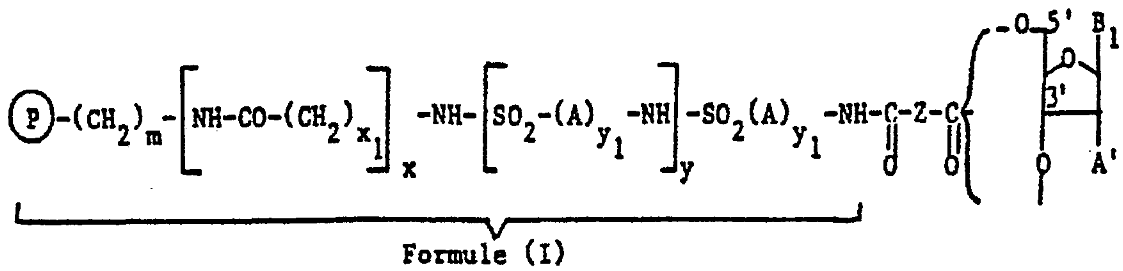

Formule (I)

formula (I) and this itself is linked via phosphodiester or triester bonds of the type:

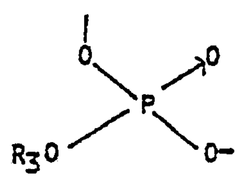

in which $R_3$ is either a hydrogen atom, or a protector group, to other nucleotides carrying the bases $B_1$, . . . . B(z–1) until the last nucleoside of formula:

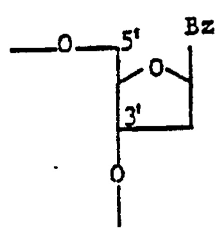

Bz being the last base of the oligodesoxy- or oligo-ribonucleotide, the hydroxy function of the last nucleoside in position 5′ or in 3′ being optionally protected and the various purine or pyrimidine bases optionally having their amine functions protected.

17. New oligodesoxyribonucleotides on supports as defined in claim 16, of formula:

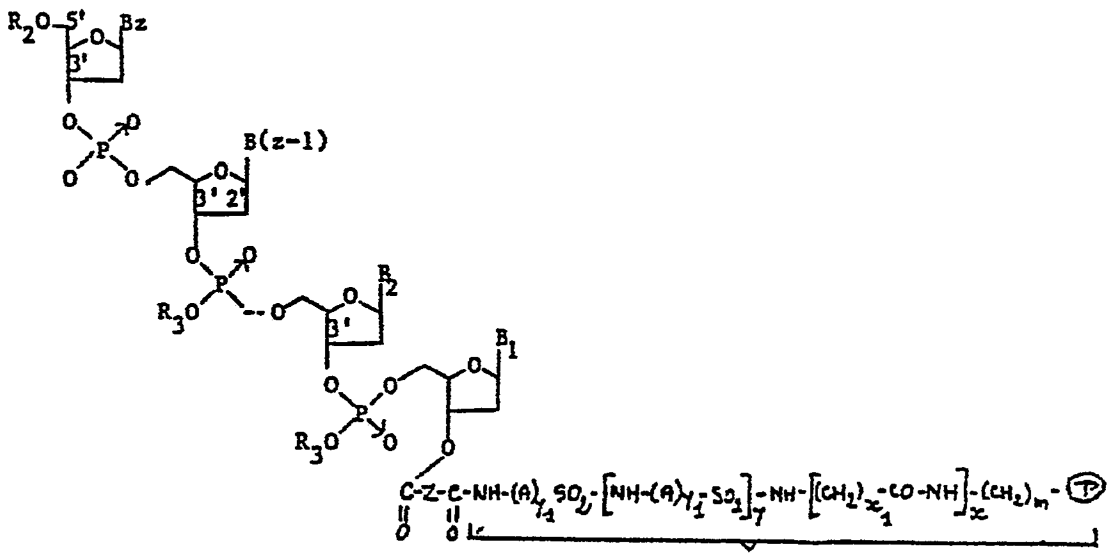

Formule (I)

in which the support of formula (I) is linked in position 3′ to an oligodesoxyribonucleotide carrying the bases $B_1$, $B_2$ ····· Bz and in which $R_2$ is either a hydrogen atom, or protector group, $R_3$ is either a hydrogen atom, or a protector group, the purine or pyrimidine bases optionally having their amine functions protected.

18. New oligodesoxyribonucleotides, oligoribonucleotides, desoxyribonucleosides, ribonucleosides on supports as defined in claims 14 to 17, in which Z is the $-(CH_2)_2-$ radical.

19. Use of supports of formula (I) as defined in any of claims 1 to 9 in the synthesis of peptides.